# EUROPÄISCHE PATENTANMELDUNG

(11) **EP 3 364 319 A1**
(43) Veröffentlichungstag der Anmeldung: **22.08.2018**
(21) Anmeldenummer: 17156330.7
(22) Anmeldetag: 15.02.2017
(51) Int. Cl.: G06F 19/00, A61M 1/00, G09B 23/28

(54) **VERFAHREN FÜR EIN SIMULATIONS- UND EVALUIERUNGSSYSTEM FÜR MEDIZINISCHE BEHANDLUNGSEINRICHTUNGEN**

(71) Anmelder: Fresenius Medical Care Deutschland GmbH, 61352 Bad Homburg (DE)
(72) Erfinder: Bluemler, Holger, 61381 Friedrichsdorf (DE); Schroers, Alexander, 60389 Frankfurt (DE); Froese, Yannick, 60433 Frankfurt (DE)
(74) Vertreter: Breuninger, Marcus

(57) **Zusammenfassung**

Die vorliegende Erfindung liegt auf dem Gebiet von Simulationssystemen für medizinische Behandlungen, insbesondere für Behandlungen, die an Dialysegeräten durchgeführt werden. Hierzu wird ein Verfahren vorgeschlagen, mit dem medizinische Behandlungen ohne die Beteiligung realer menschlicher Patienten durchgeführt und bewertet werden können.

## Beschreibung

### Technisches Gebiet

Die Erfindung liegt auf dem Gebiet von Simulationssystemen für medizinische Behandlungen, insbesondere für Behandlungen, die an Dialysegeräten durchgeführt werden.

### Hintergrund

Zur Behandlung akuter oder chronischer Erkrankungen werden häufig spezielle medizintechnische Geräte eingesetzt. Derartige medizintechnische Geräte können vielfältige Aufgaben durchführen. Um das Risiko für den Patienten in Folge von Fehlbedienungen derartiger medizintechnischer Geräte zu minimieren, werden diese i.d.R von qualifiziertem medizinischem Personal bedient. Die Schulung und die Qualifikation des medizinischen Personals sind hierbei von besonderer Bedeutung. Außerdem liegt es im Interesse der Hersteller medizintechnischer Geräte, die Geräte im Hinblick auf die Bedienbarkeit und Sicherheit zu optimieren. Um Gefahren für den Patienten auszuschließen, erfolgen die Schulung des medizinischen Personals und Untersuchungen, die die Bedienbarkeit und Sicherheit der medizintechnischen Geräte betreffen, vorteilhaft ohne einen am medizintechnischen Gerät behandelten realen menschlichen Patienten. Es stellt eine besondere Herausforderung dar, auch ohne die Beteiligung eines realen menschlichen Patienten die Schulung und die Evaluierung der Qualifikation des Bedienpersonals medizintechnischer Geräte realitätsnah durchführen zu können, sowie aussagekräftige Informationen im Hinblick auf die Bedienbarkeit und Sicherheit medizintechnischer Geräte zu erlangen.

### Zusammenfassung der Erfindung

Es besteht daher der Wunsch, eine Möglichkeit zu schaffen, das medizinische Personal zu schulen und medizintechnische Geräte im Hinblick auf die Bedienbarkeit und Sicherheit zu verbessern.

Um diesem Wunsch nachzukommen, schlägt die vorliegende Erfindung ein Verfahren nach Anspruch 1 vor.

Bevorzugte Ausführungsformen werden durch die abhängigen Ansprüche beschrieben.

Im Folgenden soll die vorliegende Erfindung am Beispiel eines Dialysegeräts als Beispiel einer Ausführungsform eines medizintechnischen Geräts dargelegt werden.

Dialysegeräte sind Blutbehandlungsgeräte, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin, deren Inhalt hiermit vollumfänglich im Offenbarungsgehalt der vorliegenden Anmeldung einbezogen wird.

Die Dialyse ist ein Verfahren zur Blutreinigung von Patienten mit akuter oder chronischer Niereninsuffizienz. Grundsätzlich unterscheidet man hierbei zwischen Verfahren mit einem extrakorporalen Blutkreislauf, wie der Hämodialyse, der Hämofiltration oder der Hämodiafiltration und der Peritonealdialyse, die keinen extrakorporalen Blutkreislauf aufweist.

Das Blut wird bei der Hämodialyse in einem extrakorporalen Blutkreislauf durch die Blutkammer eines Dialysators geleitet, die über eine semipermeable Membran von einer Dialysierflüssigkeitskammer getrennt ist. Die Dialysierflüssigkeitskammer wird von einer Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration der Blutelektrolyte im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Harnpflichtige Stoffe diffundieren durch die Membran von der Blutkammer in die Kammer für Dialysierflüssigkeit, während im Blut und in der Dialysierflüssigkeit vorhandene Elektrolyte jeweils von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser auch als Ultrafiltration bezeichnete Vorgang führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatlösung zugesetzt werden. Je nachdem, ob diese Substituatlösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von im Blut enthaltenem Plasmawasser durch einen Druckgradienten an der Membran des Dialysators.

Die Verfahren der Hämodialyse, der Hämofiltration und der Hämodiafiltration werden in der Regel mit automatischen Hämodialysegeräten durchgeführt, wie sie beispielsweise von der Anmelderin vertrieben werden.

Die Plasmapherese ist ein Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben.

Bei der Peritonealdialyse wird die Bauchhöhle eines Patienten über einen durch die Bauchdecke geführten Katheter mit einer Dialyseflüssigkeit befüllt, die ein Konzentrationsgefälle von Blutsubstanzen wie Elektrolyte (beispielsweise Natrium, Kalzium und Magnesium) gegenüber den körpereigenen Flüssigkeiten aufweist. Über das als Membran wirkende Bauchfell (Peritoneum) treten im Körper vorliegende Giftstoffe aus den im Bauchfell verlaufenden Blutgefäßen in die Bauchhöhle über. Nach einigen Stunden wird die sich in der Bauchhöhle des Patienten befindliche, nunmehr mit den aus dem Körper übergetretenen Giftstoffen versetzte Dialyseflüssigkeit ausgetauscht. Durch osmotische Vorgänge kann Wasser aus dem Blut des Patienten über das Bauchfell in die Dialyseflüssigkeit übertreten und den Patienten somit entwässern.

Das Verfahren zur Peritonealdialyse wird in der Regel mit Hilfe von automatischen Peritonealdialysegeräten, wie sie beispielsweise von der Anmelderin vertrieben werden, durchgeführt.

Dialysegeräte, als Beispiel für komplexe medizintechnische Geräte, haben umfangreiche Funktionen. Um diese Funktionen zu steuern, sind medizintechnische Geräte wie Dialysegeräte mit zumindest einem Steuergerät ausgerüstet. Diese können eine oder mehrere CPUs (central processing unit) oder Mikrokontroller umfassen, die von Softwareprogrammen gesteuert werden. Im Lichte der Offenbarung der vorliegenden Erfindung ist es nicht wesentlich, ob die beschriebenen Verfahren von einem oder mehreren Steuergeräten durchgeführt werden. Demnach gilt auch eine Vielzahl von Steuergeräten, die einzeln oder im Verbund die beschriebenen Verfahren ausführen, als Steuergerät. Die Softwareprogramme werden in der Regel in einer internen Speichervorrichtung vorgehalten. Weitere Speichervorrichtungen können zum Abspeichern sonstiger Informationen, wie Behandlungsdaten, vorgehalten werden.

Mit Dialysegeräten sind vielfältige Therapien durchführbar. Für die Vorbereitung und die Durchführung einer mit dem Dialysegerät ausgeführten Therapie sind umfangreiche Tätigkeiten des medizinischen Personals sowohl am medizintechnischen Gerät als auch am Patienten selbst notwendig. Hierfür bedarf es einer speziellen Qualifikation des medizinischen Personals, die sich einerseits auf das jeweilige Dialysegerät richtet, andererseits aber auch die Patientenüberwachung betrifft. Diese Qualifikation kann durch entsprechendes Training erworben und ausgebaut werden.

Außerdem liegt es im Interesse des Herstellers eines Dialysegeräts, die Bedienbarkeit seiner Geräte zu optimieren. Unter der Bedienbarkeit eines Geräts wird hierbei die Geräte-Bediener Interaktion im weitesten Sinne verstanden. Alle Handlungen, die am Gerät vom Bediener ausgeführt werden müssen, oder die Ausgestaltung von Meldungen, die vom Gerät an den Bediener gerichtet sind, fallen unter den Begriff Bedienbarkeit. Eine verbesserte Bedienbarkeit führt beispielsweise dazu, dass ein Bediener einen bestimmten Touchkey (Bedientaste) auf dem Touchscreen Display eines Dialysegeräts, den er bedienen möchte, oder eine bestimmte Anzeige, die er wahrnehmen möchte, schnell findet. Ebenfalls kann unter einer verbesserten Bedienbarkeit beispielsweise verstanden werden, dass bestimmte Handlungsschritte, beispielsweise das Aufrüsten von Dialysegeräten mit Einwegartikeln, wie Schlauchsets oder Dialysefilter, besser vom Bediener ausgeführt werden können.

Unabhängig davon, ob die Qualifikation des medizinischen Personals oder die Bedienbarkeit des medizintechnischen Geräts verbessert oder bewertet werden sollen (oder beides), ist es wesentlich, dass zu diesem Zweck eine konkrete medizinische Behandlung mit dem konkreten medizintechnischen Gerät durchgeführt und beobachtet wird.

Um möglichst umfassende Aussagen über die Qualifikation des medizinischen Personals und die Bedienbarkeit des medizintechnischen Geräts möglich zu machen, ist es hierbei von Vorteil, dass alle Situationen, die bei der Behandlung auftreten können, auch tatsächlich herbeigeführt werden können. Dies betrifft insbesondere auch Alarmsituationen, die ernste gesundheitliche Folgen für einen Patienten haben können. Es versteht sich daher von selbst, dass dies nicht mit realen menschlichen Patienten erfolgen sollte.

Es ist daher vorgesehen, einen künstlichen Ersatzpatienten, auch Dummy-Patient genannt, zu verwenden.

Um die Qualifikation des medizinischen Personals und/oder die Bedienbarkeit des medizintechnischen Geräts zu verbessern und/oder zu bewerten, werden die folgenden Schritte vorgeschlagen:
- Bereitstellen eines medizintechnischen Gerätes, dessen Betriebsparameter extern steuer- und beobachtbar sind.
- Bereitstellen eines künstlichen Ersatzpatienten, der mit für die zu betrachtende Behandlung wesentlichen Vorrichtungen ausgerüstet ist. Eine derartige Vorrichtung ist beispielsweise ein zumindest rudimentäres vaskulares System, wodurch der künstliche Ersatzpatient mit dem medizintechnischen Gerät zum Zweck einer Behandlungsausführung interagieren kann. Die Betriebsparameter dieses künstlichen Ersatzpatienten sind vorteilhaft ebenfalls extern steuer- und beobachtbar.

- Simulation einer medizinischen Behandlung mit dem medizintechnischen Gerät an dem künstlichen Ersatzpatienten, wobei definierte Behandlungssituationen auswählbar und herbeiführbar sind.
- Beobachten und Aufzeichnen des mit der Behandlung befassten medizinischen Personals und Analysieren der aufgezeichneten Daten.

Unter Betriebsparametern werden im Lichte der vorliegenden Erfindung Einstellungen und Messwerte verstanden, die der jeweiligen Vorrichtung zugeordnet werden können. So ist die Blutpumpenrate eines Dialysegeräts ein Betriebsparameter, genauso wie der gemessene Blutdruck oder die gemessene Bluttemperatur im extrakorporalen Blutkreislauf. Mit anderen Worten kann das Wort "Betriebsparameter" den Zustand eines jeden Teils eines Geräts oder eines Geräteverbunds, insbesondere von Aktoren, Sensoren und Steuergeräten bezeichnen. Unter dem Begriff "extrakorporaler Blutkreislauf" werden im Folgenden auch die Mittel und Vorrichtungen, insbesondere Einmalartikel, wie Schläuche, Filter, Tropfkammern etc. verstanden, die einen extrakorporalen Blutkreislauf wie weiter oben beschrieben ausbilden, unabhängig davon, ob oder mit welcher Flüssigkeit dieser gefüllt ist. Insbesondere ist auch ein ungefüllter oder mit einer medizinischen Flüssigkeit wie Dialysat, Blut oder Blutersatzflüssigkeit gefüllter extrakorporaler Blutkreislauf umfasst.

### Kurzbeschreibung der Zeichnungen

Weitere Einzelheiten und Aspekte der vorliegenden Geräte und Verfahren werden anhand von in den Zeichnungen dargestellten Ausführungsbeispielen näher beschrieben.
Die Figur 1 zeigt eine beispielhafte Gesamtansicht eines erfindungsgemäßen Systems zur Simulation einer medizinischen Behandlung an einem als Hämodialysegerät ausgeführten medizintechnischen Gerät;
die Figur 2 zeigt beispielhafte Ausführungsformen eines erfindungsgemäßen künstlichen Ersatzpatienten;
die Figur 3 zeigt eine beispielhafte Ausführungsform eines erfindungsgemäßen Flüssigkeitssystems des künstlichen Ersatzpatienten;
die Figur 4 zeigt die Darstellung einer Testperson mit mehreren bespielhaften erfindungsgemäßen Personensensoren;
die Figur 5 zeigt in einer schematischen Darstellung eine beispielhafte Ausführungsform eines erfindungsgemäßen medizintechnischen Geräts;
die Figur 6 zeigt ein beispielhaftes schematisches Ablaufdiagramm für ein erfindungsgemäßes Verfahren.

### Detaillierte Beschreibung der Ausführungsbeispiele

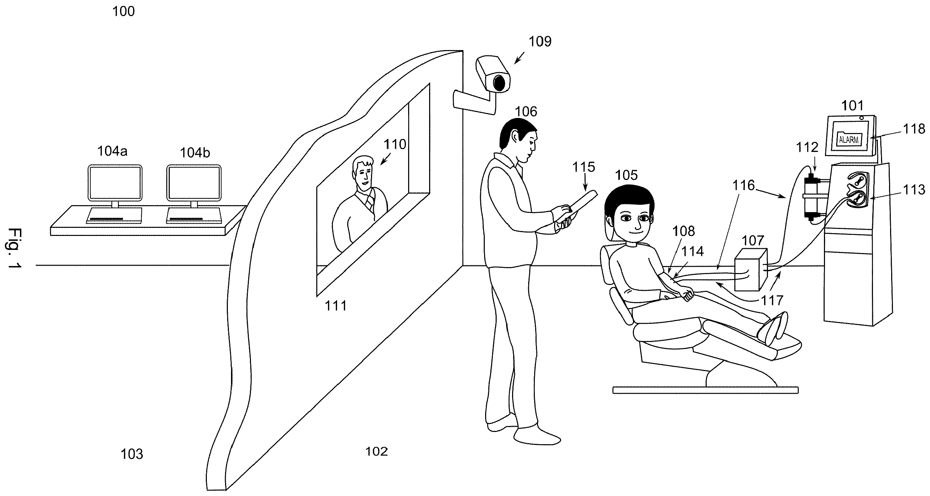

Die Figur 1 zeigt eine Gesamtansicht eines erfindungsgemäßen Systems 100 zur Simulation einer medizinischen Behandlung an einem medizintechnischen Gerät. In der in Figur 1 gezeigten Ausführungsform erstreckt sich das System über einen Beobachtungs- und Steuerungsraum 103 und einem Simulationsraum 102. Im Simulationsraum 102 befinden sich in einer Ausführungsform die an der Simulation beteiligten Vorrichtungen, insbesondere das medizintechnische Gerät 101, sowie der künstliche Ersatzpatient 105, sowie während der Simulation die Testperson 106. Der Simulationsraum 102 ist hierbei Teil der Simluationsumgebung.

Vom Beobachtungs- und Steuerungsraum 103 kann ein Beobachter 110 durch eine Öffnung 111 in einer Wand die Vorgänge im Simulationsraum 102 beobachten. Es kann vorgesehen sein, dass die Öffnung 111 von einem halbdurchlässigen Spiegel verschlossen ist, der nur in Richtung des Simulationsraums transparent ist. Im Beobachtungs- und Steuerungsraum 103 können verschiedene Steuer- und Aufzeichnungsgeräte 104a und 104b vorhanden sein, die den Ablauf der Simulation steuern und das Geschehen aufzeichnen. Für diesen Zweck sind Audio- und Videoaufnahmegeräte vorgesehen, die das Geschehen im Simulationsraum 102 aufzeichnen, wie beispielsweise die Kamera 109 in Figur 1.

Außerdem umfasst das Simulationssystem ein medizintechnisches Gerät 101, das in Figur 1 als Hämodialysegerät dargestellt ist, und einen künstlichen Ersatzpatienten 105. Der künstliche Ersatzpatient 105 kann wie in der Figur 1 eine menschliche Gestalt haben, beispielsweise in Form einer Puppe, um einen echten menschlichen Patienten möglichst realistisch zu ersetzen. Darüber hinaus kann das Simulationssystem eine oder mehrere weitere Vorrichtungen 107 umfassen, über die Einfluss auf die zu simulierende medizinische Behandlung genommen werden kann.

Über ein oder mehrere Steuergeräte 104a,b kann die medizinische Behandlung im Simulationsraum beeinflussbar sein. Dabei ist die Ausführung der Steuergeräte nebensächlich, möglich sind u.a. stationäre Computer, wie in Figur 1 dargestellt, als auch mobile Geräte wie Smartphones oder Tablet-PCs. Ein derartiges Steuergerät ist dazu konfiguriert, die simulierte Behandlung zu beeinflussen. Hierzu ist es entsprechend mit einer Software programmiert und steht in Datenkommunikation mit den an der simulierten Behandlung beteiligten Vorrichtungen. Eine Datenkommunikation kann drahtgebunden oder drahtlos erfolgen. Hierzu geeignet sind dem Fachmann bekannte Vorrichtungen und Verfahren, wie lokales Netzwerk (LAN), drahtloses lokales Netzwerk (WLAN), Bluetooth, NFC (Near Field Communication) oder sonstiges Funkkommunikationstechnologien, oder auch optische Datenübertragung beispielsweise per Infrarot.

Die Beeinflussung der Behandlung kann ablaufgesteuert sein, d.h. die Behandlung wird anhand eines vorbestimmten programmierten Ablaufs gesteuert. Eine weitere Möglichkeit ist die manuelle Steuerung über eine entsprechende Bedienerschnittstelle. Hierbei kann der Beobachter 110 jederzeit in eine laufende Behandlung eingreifen und verschiedene medizinische Situationen initiieren, auf die die Testperson 106 reagieren soll. Für eine derartige manuelle Steuerung kann eine mobile Ausführung des Steuergeräts vorgesehen sein, wodurch der Beobachter das Steuergerät mit sich führen kann und sich so in räumlicher Nähe zur Testperson bzw. zum medizintechnischen Gerät aufhalten kann.

Die Beeinflussung der Behandlung kann auch zufällig sein. Auf diese Weise weiß weder der Beobachter 110 noch die Testperson 106, welche Beeinflussung der Behandlung als nächstes von der Steuerung aufgerufen wird. Somit wird ausgeschlossen, dass die Simulation der Behandlung durch persönliche Vorlieben des Beobachters eingeschränkt oder beeinflusst wird.

Die Testperson 106, die beispielsweise ein Dialysepfleger oder ein Dialysearzt sein kann, kann einen mobilen Computer 115 mit sich führen, der als Ein-/Ausgabemöglichkeit für Informationen, die die simulierte medizinische Behandlung betreffen, dienen kann. Als Testperson kommt aber beispielsweise auch ein Entwickler von medizintechnischen Geräten in Frage.

Das in Figur 1 vereinfacht dargestellte Hämodialysegerät umfasst eine Bedienerschnittstelle 118, beispielsweise als Touchscreen Display ausgeführt, sowie zumindest ein Pumpmittel 113, mit dem Blut bzw. eine medizinische Flüssigkeit in einem extrakorporalen Blutkreislauf gepumpt werden kann. Der extrakorporale Blutkreislauf der Simulationsumgebung umfasst einen arteriellen Blutschlauch 116 und einen venösen Blutschlauch 117, sowie einen Dialysefilter 112. Der arterielle und der venöse Blutschlauch 116 und 117 werden an der Punktionsstelle 114 mit einem Flüssigkeitssystem im Arm 108 des künstlichen Ersatzpatienten verbunden. Das Flüssigkeitssystem im Arm 108 des künstlichen Ersatzpatienten umfasst weitere Vorrichtungen (in Figur 1 nicht dargestellt), die Einfluss auf den extrakorporalen Blutkreislauf haben. So können Mittel vorgesehen sein, mit denen sowohl der Flüssigkeitsdruck als auch der Fluss im arteriellen bzw. im venösen Blutschlauch 116 und 117 beeinflussbar sind.

Von der Pumpvorrichtung 113 wird Ersatzflüssigkeit für menschliches Blut, als Ausführungsform für eine medizinische Flüssigkeit, durch den Dialysefilter 112 gepumpt. In einer Ausführungsform fließt die Ersatzflüssigkeit durch eine oder mehrere zusätzliche Vorrichtungen 107, die Einfluss auf den extrakorporalen Blutkreislauf haben können.

Erfindungsgemäß ist das Gesamtsystem aus Figur 1 zum Ausführen eines Verfahrens zur Simulation einer medizinischen Behandlung eines Patienten an einem medizintechnischen Gerät konfiguriert und programmiert.

Im Einzelnen umfasst das Verfahren mehrere Aspekte:
Der Testperson 106 kann eine Aufgabe im Zusammenhang mit der Behandlung oder dem medizintechnischen Gerät gestellt werden. Diese Aufgabe kann beispielsweise lauten, das medizintechnische Gerät für eine bestimmte Behandlung betriebsfertig zu machen. Dies umfasst für gewöhnlich das Ausrüsten des medizintechnischen Geräts mit Einmalartikeln, wie am Beispiel des Hämodialysegeräts in Figur 1 dem Dialysefilter, dem Blutschlauchsystem und den Dialysierflüssigkeitsleitungen (nicht gezeigt in Figur 1). Weiterhin kann eine Bediener-Geräteinteraktion an der Bedienerschnittstelle 118 über die dort angebotene Bedienerführung notwendig sein.

Die Testperson wird bei der Bewältigung der gestellten Aufgabe beobachtet. Zur Überwachung der Testperson können eine Vielzahl von Sensoren eingesetzt werden, die im Folgenden mit dem Oberbegriff Personensensoren bezeichnet werden. Diese Beobachtung kann eine Kameraüberwachung (ggf. inklusive Tonüberwachung) umfassen. Darüber hinaus können auch weitere physiologische Parameter der Testperson überwacht werden. Beispielsweise kann der Puls der Testperson über einen entsprechenden Sensor (bspw. eine Pulsuhr) überwacht werden. Darüber hinaus können auch die Hautleitfähigkeit, die genaue Herztätigkeit (EKG) die Hirnströme (EEG) oder auch die Augenbewegung überwacht werden. Hierzu werden dem Fachmann bekannte Sensoren verwendet, deren Messwerte eindeutig einer bestimmten Aktion zugeordnet werden können, beispielsweise indem die Videoaufzeichnung und die Sensormesswerte mit eindeutigen Zeitwerten verknüpft werden (Zeitstempel).

Für die Bestimmung der Augenbewegung können sogenannte Eye-Tracker Vorrichtungen verwendet werden, die beispielsweise über eine Videoaufzeichnung der Augen der Testperson und in Blickrichtung der Testperson evaluieren, wohin die Testperson aktuell gerade schaut.

Die durch die Überwachung der Testperson gewonnenen Daten können im Hinblick auf die Qualifikation und die Bedienbarkeit und Sicherheit des medizintechnischen Geräts hin analysiert werden.

So kann beispielsweise der Erregungsgrad der Testperson über die Personensensoren bestimmt werden. Hierzu geeignete Sensoren messen beispielsweise die Herztätigkeit, Hirnströme und die Hautleitfähigkeit der Testperson. Ein schnellerer Herzschlag, eine erhöhte Hautleitfähigkeit in Folge von übermäßigem Schwitzen oder spezifische Hirnströme lassen auf einen erhöhten Erregungsgrad (Stress) schließen. In Verbindung mit der Videoaufzeichnung der Testperson kann beispielsweise so auf bestimmte Defizite in der Qualifikation bzw. auf einen erhöhten Trainingsbedarf für bestimmte, der Stresssituation zugrunde liegende Handlungen geschlossen werden.

Über die Aufzeichnung der Blickrichtung der Testperson können beispielsweise Rückschlüsse auf die Bedienbarkeit des medizintechnischen Geräts getätigt werden. Hektische Augenbewegungen, die relevante Bedienpunkte des medizintechnischen Geräts nicht nach adäquat kurzer Zeit anvisieren, können Rückschlüsse auf eine verbesserungswürdige Bedienstruktur des medizintechnischen Geräts erlauben. Muss der Bediener beispielsweise zum Auffinden eines bestimmten Menüpunkts auf der Bedienerschnittstelle erst lange suchen, gibt das dem Geräteentwickler einen Hinweis darauf, dass die Bedienerführung noch Verbesserungspotential hat. Dies ist insbesondere dann relevant, wenn die Testperson mit einer Vorgängerversion des zu untersuchenden medizintechnischen Geräts vertraut ist und eine Weiterentwicklung im Hinblick auf die Bedienbarkeit untersucht werden soll.

Die Aufgabenstellung für die Testperson kann aber auch die Durchführung einer simulierten Behandlung sein. Eine derartige Behandlung schließt sich i.d.R. an den oben beschriebenen Vorgang der Aufrüstung des medizinischen Geräts an. Sie umfasst Handlungen, die an dem künstlichen Ersatzpatienten 105 durchzuführen sind, um eine Interaktion zwischen künstlichem Ersatzpatient 105 und dem medizintechnischen Gerät, also beispielsweise einem Hämodialysegerät 101 zu ermöglichen.

Konkret am Beispiel der Figur 1 bedeutet dies, dass der arterielle und der venöse Blutschlauch 116 und 117 an der Punktionsstelle 114 mit dem Flüssigkeitssystem im Arm 108 des künstlichen Ersatzpatienten verbunden werden müssen. Das Dialysegerät ist entsprechend der zu simulierenden Behandlung aufzurüsten und einzustellen. In diesem Zusammenhang, wird die Testperson analog einer echten Behandlung beispielsweise den extrakorporalen Blutkreislauf füllen und spülen. Die Blutersatzflüssigkeit wird in einem gesonderten Behältnis vorgehalten.

In einer Ausführungsform können eine oder mehrere externe Vorrichtungen 107, die Einfluss auf die zu simulierende medizinische Behandlung nehmen können, angeschlossen werden oder schon angeschlossen sein. Im zweiten Fall kann eine Fluidleitung zwischen künstlichem Ersatzpatienten 105 und der externen Vorrichtung 107 schon vorliegen, die Testperson stellt dementsprechend eine Fluidleitung zwischen der Vorrichtung 107 und dem Dialysegerät 101 her.

Der Einfluss der externen Vorrichtung 107 auf die zu simulierende medizinische Behandlung kann beispielsweise umfassen, den Flüssigkeitsfluss durch die arterielle bzw. venöse Leitung zu vermindern, zu stoppen oder umzuleiten. Dies simuliert ein teilweises oder vollständiges Abknicken der entsprechenden Leitung, bzw. eine Unterbrechung des Flüssigkeitskreislaufs beispielsweise durch ein Herausrutschen einer Nadel an der Punktionsstelle. Es kann aber auch vorgesehen sein, dass die technischen Mittel der Vorrichtung 107 innerhalb des künstlichen Ersatzpatienten angeordnet sind. Dies erhöht den Realismus der simulierten Behandlung.

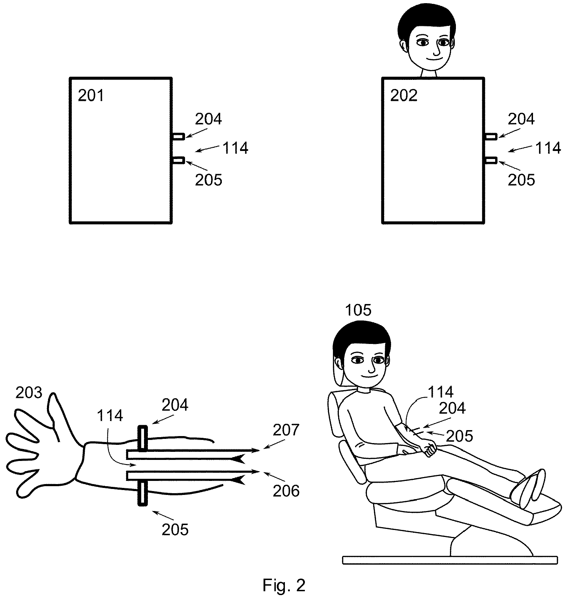

Die Figur 2 zeigt Ausführungsformen des künstlichen Ersatzpatienten. Hierbei unterscheiden sich die einzelnen Ausführungsformen im Grad des Realismus des künstlichen Ersatzpatienten im Hinblick auf einen menschlichen Patienten. Der künstliche Ersatzpatient dient in seinen Ausführungsformen als Vorrichtung zur Nachbildung verschiedenster menschlichen Eigenschafen.

Diese menschlichen Eigenschaften können sowohl körperliche Eigenschaften umfassen, wie beispielsweise Alter, Geschlecht, Gewicht, Rasse, Größe etc., oder auch das Aussehen der Augen (Augenbild). Weiterhin umfasst der Begriff menschliche Eigenschaften auch momentane Zustände von Körperteilen, Organen oder Körperflüssigkeiten, wie beispielsweise ausgeführte Bewegung von Teilen des menschlichen Körpers, Zustand des menschlichen Puls, Viskosität der Flüssigkeit im vaskulären System, Temperatur der Flüssigkeit im vaskulären System, Zustand der Atmung etc..

Weitere menschliche Eigenschaften betreffen unter anderem auch den Geisteszustand, das momentane Wohlbefinden oder auch menschliche Lautäußerungen.

Kurz gesagt umfasst der Begriff "menschliche Eigenschaften" insbesondere alle mit Eigenschaften eines realen Patienten, die für die Behandlung des Patienten an dem medizintechnischen Gerät von Relevanz sein können.

Im einfachsten Fall besteht zwischen einem menschlichen Patient und einem künstlichen Ersatzpatient keine Ähnlichkeit. Für ein System zur Simulation einer Dialysebehandlung, die beispielsweise den Fokus auf die Bedienbarkeit des an der Simulation beteiligten Dialysegeräts legt, muss der künstliche Ersatzpatient keinerlei Ähnlichkeit mit einem echten Menschen aufweisen. So weist die Ausführung 201 des künstlichen Ersatzpatienten nur die Punktionsstelle 114 mit den Fluidleitungen 204 und 205 auf, die die arterielle (204) und venöse (205) Zugangsstelle zum simulierten vaskulären System des Ersatzpatienten realisieren. Diese Fluidleitungen können beispielsweise medizinische Luer-Lock Verbinder aufweisen, an die der extrakorporale Blutkreislauf, der entsprechende Gegenstücke aufweist, angeschlossen werden kann.

Die Ausführung 202 des künstlichen Ersatzpatienten weist zusätzlich eine Nachbildung eines menschlichen Kopfs auf, der weitere Vorrichtungen aufweisen kann, um reales menschliches Verhalten bzw. Reaktionen nachbilden zu können.

Eine weitere Ausführung des künstlichen Ersatzpatienten ist als Nachbildung eines menschlichen Arms 203 zu sehen. Auch dieser weist eine Punktionsstelle 114 auf und kann wie die Ausführung 201 mit Fluidleitungen 204 und 205 ausgestattet sein. In der Armnachbildung 203 verlaufen Fluidleitungen 206 und 207, die das reale vaskuläre System eines Menschen nachstellen. In einer Ausführungsform der Armnachbildung kann Flüssigkeit über einen dafür vorgesehenen Fluidanschluss (beispielsweise 204) in den Arm hineinlaufen und Flüssigkeit über einen dafür vorgesehenen Fluidanschluss (beispielsweise 205) aus dem Arm herauslaufen. Die Flüsse und die Drücke in den Fluidleitungen 206 und 207 können unabhängig voneinander einstellbar sein. Dies ist in Figur 2 dadurch angedeutet, dass sowohl die Fluidleitung 206 als auch die Fluidleitung 207 einen Zulauf und einen Ablauf besitzen. Diese Fluidleitungen können durch unabhängige Vorrichtungen, wie Pumpen, Fluidreservoire, Ventile (nicht in Figur 2 dargestellt) etc. beeinflussbar sein.

Die Ausführung 105 zeigt schließlich einen künstlichen Ersatzpatienten, der in seiner Gestalt einem realen menschlichen Patienten am ähnlichsten ist.

Sinn dieser verschiedenen Ausführungsformen des künstlichen Ersatzpatienten ist es, die Komplexität und die Ausstattung des künstlichen Ersatzpatienten an das Ziel der Simulation anzupassen. Somit kann unter Berücksichtigung der Kosten sichergestellt werden, dass der künstliche Ersatzpatient die für die Zielsetzung der Simulation mindestens notwendigen Eigenschaften aufweist. Eine hypothetisch perfekte Nachbildung eines realen Patienten wäre im Hinblick auf den Realismus ideal, würde aber die Kosten für eine Simulationsaufgabe, die diesen Realismus nicht erfordert, unnötig erhöhen.

Der künstliche Ersatzpatient kann folgende Merkmale aufweisen:
Der künstliche Ersatzpatient kann als Puppe von normaler menschlicher Statur ausgeführt sein.

Im Hinblick auf die Qualifizierung des medizinischen Personals für die Behandlung besonderer Patientengruppen kann der künstliche Ersatzpatient in seiner Ausführung Merkmale dieser Gruppe aufweisen. Z.B. kann der künstliche Ersatzpatient in seiner Statur Merkmale älterer Patienten, dementer Patienten, adipöse Patienten oder kindlicher Patienten aufweisen. Die äußeren Merkmale betreffen insbesondere die Statur und das Aussehen des künstlichen Ersatzpatienten, während das Gesamtsystem dazu eingerichtet sein kann, die speziellen Eigenschaften einer Patientengruppe zu simulieren. Diese insbesondere von der Steuerung des künstlichen Ersatzpatienten definierten Eigenschaften betreffen die medizinischen, physiologischen und psychologischen Besonderheiten der jeweiligen Patientengruppe. So kann das reale Verhalten einer Patientengruppe durch die Ansteuerung der Vorrichtungen des künstlichen Ersatzpatienten weitestgehend nachgestellt werden.

Eine weitere Möglichkeit, reale Patienten möglichst exakt zu simulieren und die Qualität der Qualifizierung des medizinischen Personals zu steigern, besteht darin, den künstlichen Ersatzpatienten mit künstlichen Augen auszustatten. Hierdurch wird es ermöglicht, das medizinische Personal im Hinblick auf die Augendiagnostik zu schulen. Hintergrund einer derartigen Augensimulation ist, dass bestimmte Umstände, die bei einer realen Behandlung auftreten können, sich in dem Augenbild des Patienten widerspiegeln können.

Zum einen könnte ein wacher von einem schlafenden Patient unterschieden werden, in dem die Augen einerseits geöffnet und andererseits mit geschlossenen Augenlidern dargestellt werden. Des Weiteren könnte ein theatralisches Augenverdrehen im Zusammenspiel mit einem anderen Parameter, z.B. fallender Blutdruck, auf eine drohende Ohnmacht des Patienten hinweisen. Das Augenbild kann auch Unruhe, Angst oder Müdigkeit andeuten.

Der Kopf des künstlichen Ersatzpatienten kann zum Zweck der Augensimulation mit kleinen Monitoren ausgestattet sein, die in den Augenöffnungen platziert sein können. Eine mögliche andere Ausführungsform umfasst eine Brille, die dem künstlichen Ersatzpatienten aufgesetzt werden kann, wobei die Brillengläser Monitore sind. Die Monitore werden derart angesteuert, dass sie die Abbildung von menschlichen Augen darstellen. Diese Abbildungen können animiert sein bzw. können Videosequenzen umfassen, um die Veränderungen der Darstellung menschlicher Augen möglichst realitätsnah zu simulieren. In einer Ausführungsform sind die Monitore OLED Displays. OLED Displays bieten einen großen Betrachtungswinkel, so dass das medizinische Personal von jedem Standort aus die simulierten Augen gut betrachten kann. Weiterhin bietet die OLED Display Technologie die Möglichkeit, gewölbte Monitore zu erschaffen, um einen realen Augapfel möglichst realitätsnah zu simulieren. Eine weitere Ausführungsform für die Augensimulation sieht vor, das Augenbild zu projizieren.

Eine weitere Ausgestaltung des künstlichen Patienten sieht vor, dass dieser mit einem Lautsprecher ausgerüstet ist. Dieser kann zur Simulation von Lautäußerungen eines realen Patienten verwendet werden. Dem künstlichen Patienten wird somit quasi eine "Stimme" verliehen. Während der Simulation einer medizinischen Behandlung können über den Lautsprecher menschliche Äußerungen bzw. Laute ausgegeben werden. Diese können beispielsweise Bedürfnisse wie Hunger, Durst oder einen notwendigen Toilettengang des simulierten Patienten betreffen, oder auch Ausdruck des medizinischen Zustands sein, beispielsweise Atemgeräusche oder Stöhnen. Das medizinische Personal muss entsprechend der jeweiligen Äußerung reagieren und gegebenenfalls bestimmte Handlungen am künstlichen Ersatzpatienten durchführen.

In einer Ausführungsform kann auch eine verbale Interaktion zwischen künstlichen Ersatzpatienten und dem medizinischen Personal bewertet werden. Beispielsweise kann es vorgesehen sein, dass das medizinische Personal auf bestimmte Äußerungen des künstlichen Ersatzpatienten im Idealfall bestimmte Antworten oder gezielte Nachfragen geben sollte. Beispielsweise kann es vorgesehen sein, dass auf ein Jammern oder Stöhnen, das der künstliche Ersatzpatient über einen Lautsprecher ausgibt, die Nachfrage nach dem Befinden vom medizinischen Personal erfolgen sollte. Die Äußerungen des medizinischen Personals können über Mikrofone aufgezeichnet werden. Bei der Bewertung dieser Äußerungen kann auf die Technologie der Spracherkennung zurückgegriffen werden. In einer weiteren Ausführungsform kann aber auch eine weitere Person die Äußerungen bewerten.

Generell können die Lautäußerungen des künstlichen Ersatzpatienten ablaufgesteuert ausgegeben werden, d.h. sie liegen als Datei abgespeichert vor und werden zu bestimmten Zeitpunkten ausgegeben. Lautäußerungen können aber auch zufällig sowohl die Art als auch den Zeitpunkt der Lautäußerung betreffend ausgegeben werden.

Es kann aber auch vorgesehen sein, dass die Äußerungen über ein Mikrofon beispielsweise aus dem Überwachungsraum von einer realen Person, beispielsweise einem Ausbilder oder einem Dialysearzt eingesprochen und über den Lautsprecher des künstlichen Ersatzpatienten ausgegeben werden. Auf diese Weise kann flexibel auf die Gesamtsituation während der Simulation reagiert werden.

Lautäußerungen des künstlichen Patienten können mit der Steuerung weiterer Mittel des künstlichen Patienten gekoppelt werden. So können beispielsweise Laute, die auf ein Unwohlsein schließen lassen sollen, damit verknüpft werden, dass die Mittel, die den Flüssigkeitsdrück im simulierten vaskulären System des künstlichen Ersatzpatienten beeinflussen, derart angesteuert werden, dass ein entgleisender Blutdruck simuliert wird.

Über die Lautmeldungen kann dem künstlichen Ersatzpatienten ein für eine bestimmte Patientengruppe typischer Charakter zugewiesen werden, in dem charakteristische Lautäußerungen abgespielt werden. So kann beispielsweise ein dementer Patient simuliert werden.

Demente Patienten sind eine wachsende Herausforderung im Dialyseumfeld. Schließt die trainierende Testperson aufgrund der charakteristischen Lautäußerungen des künstlichen Ersatzpatienten beispielsweise auf einen dementen Patienten, sollte er verstärkt auf ein unruhiges Verhalten des Patienten achten, möglicherweise gefolgt von einem Nadelverlust, also dem Herausrutschen einer der Nadeln bzw. medizinischer Verbinder, die den extrakorporalen Blutkreislauf mit dem (simulierten) vaskulären System des künstlichen Ersatzpatienten verbinden. Dies kann durch die Überwachung der trainierenden Testperson wie schon zuvor beschrieben evaluiert werden.

Mit dem Wissen um den besonderen Charakter des simulierten Ersatzpatienten kann die trainierende Testperson auch die Kommunikation zwischen ihm und dem künstlichen Ersatzpatienten anpassen. So können Anzahl und Art der Nachfragen der trainierenden Testperson an den künstlichen Ersatzpatienten je nach dessen Konfiguration, d.h. nach der Patientengruppe, die er simulieren soll, bewertet werden. So bedürfen Kinder beispielsweise einer anderen Art der Kommunikation als demente Patienten. Die verbalen an den künstlichen Patienten gerichteten Lautäußerungen der trainierenden Testperson können entsprechend aufgezeichnet und bewertet werden, wobei diese Bewertung im Hinblick auf die Qualifikation der trainierenden Testperson einfließen kann.

Generell können die verbalen Äußerungen des künstlichen Ersatzpatienten in einer beliebigen Sprache ausgegeben werden. Somit können trainierende Testpersonen beliebiger Nationalität an dem Simulationssystem üben. Darüber hinaus kann somit auch gegebenenfalls die Fremdsprachenfähigkeit der Testperson evaluiert bzw. trainiert werden.

Bei der Hämodialyse von Akutpatienten wird i.d.R. das vaskuläre System des Patienten über einen Halskatheter angeschlossen. Es kann vorgesehen sein, dass ein entsprechender Anschluss am künstlichen Patienten vorhanden ist.

Der künstliche Ersatzpatient kann zur Simulation der Atembewegung Aktoren aufweisen. Hierdurch kann, gegebenenfalls auch in Verbindung mit dem Lautsprecher, die Atmung simuliert werden, in dem die Aktoren derart angesteuert werden, dass sich beispielsweise der Brustkorb periodisch hebt und senkt. Entsprechende Atemgeräusche können die Simulation der Atemtätigkeit noch realitätsnäher gestalten.

Die Atmungssimulation lässt sich sowohl mit dem angenommenen emotionalen Erregungszustand, als auch mit der medizinischen Situation des künstlichen Ersatzpatienten in Verbindung bringen. Z.B. kann die Atmungssimulation derart angesteuert werden, dass Schlaf oder Angst des künstlichen Ersatzpatienten simuliert werden. Dies ist besonders vorteilhaft mit der Augensimulation verknüpft: geschlossene Augen und regelmäßiger tiefer Atem deuten auf Schlaf, aufgerissene Augen und unregelmäßiger flacher Atem deuten auf Angst hin. Ein simulierter Blutdruckabfall dagegen in Verbindung mit schwacher simulierter Atmung deutet auf ein kardiovaskuläres Problem hin.

In einer weiteren Ausführungsform kann der künstliche Ersatzpatient über Aktoren zur Simulation eines Pulses verfügen. Die Simulation eines Herzschlages kann beispielsweise über den gepulsten Betrieb von Flüssigkeitspumpen erfolgen, die die Blutersatzflüssigkeit im simulierten vaskulären System des künstlichen Patienten fördern. Ebenfalls sind auch entsprechend angesteuerte Durchflussdrosseln denkbar. Es kann weiterhin vorgesehen sein, dass ein mechanischer Aktor, beispielsweise ein Hub-Magnet, einen fühlbaren Puls erzeugt.

Weiterhin kann vorgesehen sein, dass ein hörbarer Herzschlag innerhalb des künstlichen Ersatzpatienten erzeugt wird. Der so erzeugte Puls kann in schon beschriebener Weise gesteuert werden, um verschiedene medizinische Indikationen bzw. Patientenzustände zu simulieren.

In einer weiteren Ausführungsform kann es vorgesehen sein, dass Blutdruckmessungen, die das medizinische Personal am Arm des künstlichen Ersatzpatienten durchführen kann, beeinflussbar sind. Beispielsweise kann ein Lautsprecher in die Blutdruckmanschette eingebaut sein, der das Ergebnis der Messung bestimmt. Ein so gemessener niedriger Blutdruck würde dazu animieren, blutdruckhebende Maßnahmen, wie z.B. Änderungen an der Ultrafiltrationsrate, durchzuführen und vielleicht in kürzeren Zeitabständen den Erfolg dieser Maßnahme zu kontrollieren. Eine so initiierte Gegenmaßnahme kann dazu führen, dass sich der simulierte Blutdruck wieder normalisiert. Dementsprechend werden die Mittel zur Beeinflussung des Blutdrucks im vaskulären System des künstlichen Ersatzpatienten angesteuert, bzw. werden auch die Mittel zur Beeinflussung der Messung des Blutdrucks, hier also der Lautsprecher in einer Blutdruckmanschette entsprechend angesteuert, um eine Normalisierung des Blutdrucks für das medizinische Personal anzuzeigen.

In einer weiteren Ausführungsform kann es vorgesehen sein, dass der künstliche Ersatzpatient reale Bewegungen vollführt. Hierzu kann er mit entsprechenden Aktoren wie beispielsweise Elektromotoren, die über entsprechende Mechaniken beispielsweise Gliedmaßen bewegen, ausgerüstet sein. Alternativ kann auch der Patientensessel gesteuert werden, beispielsweise kann die Rückenlehne oder eine Beinstütze motorisch bewegt werden. Durch die Patientenbewegung kann beispielsweise ein unruhiger Patient simuliert werden. Bedingt durch die Bewegung des künstlichen Ersatzpatienten kann beispielsweise ein realer Nadelverlust oder eine echte Schlauchknicksituation erzeugt werden. Auch die simulierte Patientenbewegung kann mit anderen Vorrichtungen des künstlichen Ersatzpatienten gekoppelt werden, beispielsweise mit den Mitteln zur Pulserzeugung oder mit den Mitteln zur Lauterzeugung.

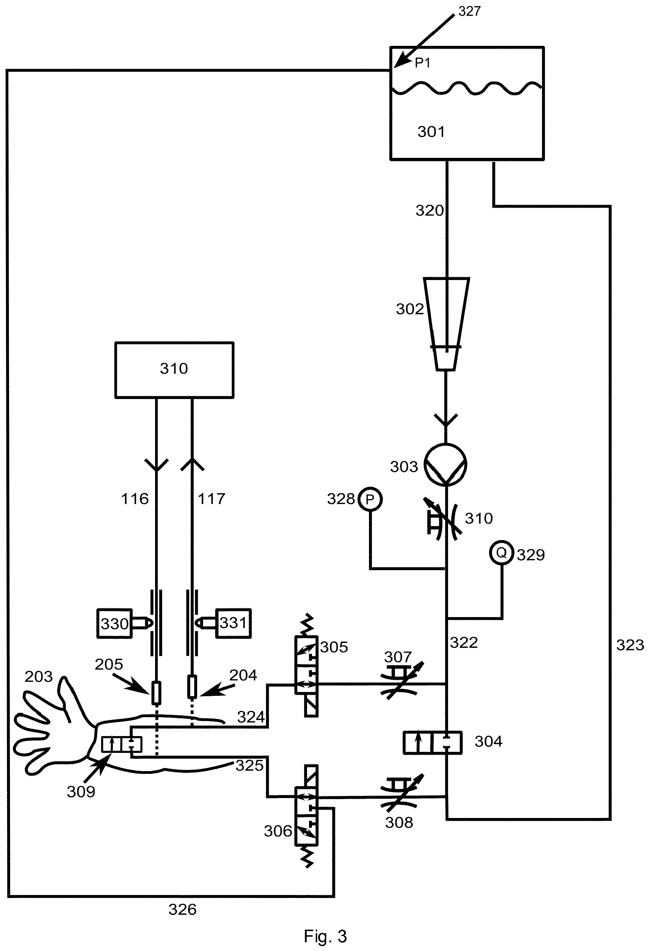

Die Figur 3 zeigt in einer detaillierteren Darstellung eine Ausführungsform des simulierten vaskulären Systems des künstlichen Ersatzpatienten mit Teilen des extrakorporalen Blutkreislaufs.

In der Figur 3 ergibt sich bei geöffnetem Ventil 304 ein erster Fluidkreislauf, der gebildet wird aus den Elementen Reservoir 301, Fluidleitung 320, Tropfkammer 302, Pumpe 303, Drosselventil 310, Fluidleitung 322, Ventil 304 und Fluidleitung 323.

Das Drosselventil 310 ermöglicht es, den Flüssigkeitsfluss und den Flüssigkeitsdruck unabhängig von der Pumpe 303 zu verändern.

Innerhalb des Arms des künstlichen Ersatzpatienten verlaufen die Fluidleitungen 324, die einer Arterie eines echten Patienten entspricht, sowie die Fluidleitung 325, die einer Vene eines echten Patienten entspricht.

Die beiden Fluidleitungen 324 und 325 können über das Ventil 309 miteinander verbunden werden.

Das Ventil 304 leitet im Normalbetrieb und wird nur für das Befüllen des Gesamtkreislaufs gesperrt. Beim Befüllen wird entsprechend das Ventil 309 geöffnet, so dass die Fluidleitungen 324 und 325 miteinander verbunden sind. In dieser Konfiguration kann das gesamte Fluidsystem des künstlichen Arms 203 mit Blutersatzflüssigkeit, beispielsweise aus dem Reservoir 301 befüllt werden. Im Normalbetrieb ist das Ventil 309 gesperrt.

Die beiden Fluidleitungen 324 und 325 sind weiterhin über die 3-Wegeventil 305 und 306 und über die Proportionalventile 307 und 308 (Drosselventile) mit dem ersten Flüssigkeitskreislauf (Fluidleitungen 322 und 323) verbunden. Über die einzeln steuerbaren Proportionalventile 307 und 308 kann der Flüssigkeitsfluss und der Flüssigkeitsdruck innerhalb der Fluidleitungen 324 und 325 einzeln beeinflusst werden.

Das 3-Wegeventil 305 kann die Fluidleitung 324 entweder mit der Fluidleitungen 322 oder mit der Außenluft verbinden.

Das 3-Wegeventil 306 kann die Fluidleitung 325 entweder mit der Fluidleitung 323 oder mit der Fluidleitung 326 verbinden, welche zum Überlauf des Reservoirs 301 führt.

An der Punktionsstelle können die Fluidleitungen 324 und 325 nach außen hin mit dem extrakorporalen Blutkreislauf einer Dialysemaschine 310 verbunden werden. Die Verbindung kann beispielsweise wie schon in Figur 1 gezeigt über medizinische Luer-Lockverbinder 204 und 205 hergestellt werden, die zum vaskulären System des künstlichen Ersatzpatienten mittels Hohlnadeln (in Figur 3 gestrichelt gezeichnet) eine Fluidverbindung herstellen können.

Ein in der Figur 3 nicht gezeigte Blutpumpe des Dialysegeräts 310 kann zusätzlich die Flüsse und Fluiddrücke in den einzelnen Fluidverbindern beeinflussen. Sämtliche Flüsse und Fluiddrücke können über entsprechende Sensoren, wie exemplarisch in Figur 3 der Drucksensor 328 und der Flusssensor 329, überwacht werden.

Die beiden Abquetschvorrichtungen 330 und 331 dienen dazu, die Fluidleitungen 116 und 117 gezielt zu beeinflussen, um beispielsweise ein Abknicken der Blutschläuche zu simulieren. Eine derartige Abquetschvorrichtung kann einen verfahrbaren Kolben aufweisen, der einen verformbaren Schlauch fluiddicht abquetscht oder den Durchfluss durch diesen Schlauch behindert. Eine alternative Abquetschvorrichtung ist beispielsweise eine ansteuerbare Schlauchklemme.

Die Aktoren und Sensoren des in Figur 3 dargestellten Systems sind ansteuer- bzw. auslesbar. Hierzu sind sie mit zumindest einem Steuergerät datentechnisch verbunden (nicht in Figur 3 dargestellt), das Steuerbefehle an die Aktoren sendet und Sensordaten von den Sensoren empfangen kann.

Durch eine derartige Ansteuerung sind vielfältige medizinische Situationen simulierbar.

Soll beispielsweise ein arterieller Nadelverlust simuliert werden, also das Herausrutschen der arteriellen Nadel aus der Punktionsstelle, können die folgenden Schritte vom Steuergerät initiiert werden:
Das 3-Wegeventil 305 wird vom Steuergerät derart angesteuert, dass es die Fluidleitung 324 mit der Außenluft verbindet, worauf durch das Saugen der Blutpumpe des Dialysegeräts Luft in den Kreislauf und somit in den arteriellen Zugang eindringt. Ein für Dialysegeräte im Allgemeinen gebräuchlicher Sensor zum Bestimmen des Fluiddrucks vor der Blutpumpe misst in der Folge entsprechend eine Druckveränderung innerhalb des Zugangsschlauchs (in Figur 3 Fluidleitung 117), da die Fluidleitung 117 nicht mehr mit der als Druckquelle fungierenden Pumpe 303 verbunden ist, sondern über das 3-Wegeventil 305 mit dem atmosphärischen Außendruck.

Dialysegeräte sind zur Überwachung der Behandlung und zur Gewährleistung der Patientensicherheit i.d.R. mit vielfältigen Sicherheitsvorrichtungen ausgerüstet. So kann beispielsweise eine gemessene plötzliche Druckveränderung innerhalb einer Zugangsleitung (117 oder 116) von einer Sicherheitsvorrichtung des Dialysegeräts als Nadelverlust interpretiert werden, woraufhin das Dialysegerät in einen sicheren Betriebsmodus überführt wird, beispielsweise durch Absperren des extrakorporalen Blutkreislaufs, Stoppen der Blutpumpe und entsprechender Alarmmeldung auf einer Bedienerschnittstelle.

Ein weiteres Indiz für einen Nadelverlust, insbesondere für einen Verlust der arteriellen Nadel, kann ein Absinken des Blutpegels in der venösen Tropfkammer des Dialysegeräts sein (nicht dargestellt in Figur 3), welches durch entsprechende Pegelsensoren erkannt werden kann.

Soll ein venöser Nadelverlust simuliert werden, wird das 3-Wegeventil 306 vom Steuergerät derart angesteuert, dass es die Fluidleitung 325 mit der Fluidleitung 326 verbindet, woraufhin Blutersatzflüssigkeit von der Blutpumpe des Dialysegeräts in das Reservoir 301 gepumpt wird. Bei einem realen venösen Nadelverlust würde ohne weitere Maßnahme Blut des Patienten von der Blutpumpe des Dialysegeräts ins Freie gelangen. Bei der Simulation wird in der Ausführungsform nach der Figur 3 verhindert, dass die Blutersatzflüssigkeit unkontrolliert ins Freie gelangt, in dem das 3-Wegeventil 306 die Fluidverbindung zwischen den Fluidleitungen 325 und 326 herstellt, wodurch die Blutersatzflüssigkeit in das Reservoir 301 gepumpt wird. Der Druck P1 im Reservoir 301 am Überlauf 327 entspricht dem atmosphärischen Außendruck. Das Reservoir 301 kann zur Gewährleistung dieses Druckes an dieser Stelle beispielsweise mit einer Öffnung nach außen ausgerüstet sein.

Eine Sicherheitsvorrichtung des Dialysegeräts misst somit im Falle des simulierten venösen Nadelverlusts den Druck am Austritt des Überlaufs, was einer sich an Luft befindlichen Nadel entspricht, und löst die schon beschriebenen Folgeaktionen aus, beispielsweise Absperren des extrakorporalen Blutkreislaufs, Stopp der Blutpumpe, entsprechende Alarmmeldung auf einer Bedienerschnittstelle.

Ein weitere kritische Situation, die während einer Dialysebehandlung auftreten kann betrifft das Ansaugen der arteriellen Nadel an der Gefäßwand des punktierten Gefäßes (bei chronischer Dialyse i.d.R. eine Fistel).

Ein Ansaugen der arteriellen Nadel in der Realität hat einen sinkenden Blutfluss und einen starken Unterdruck in der arteriellen Zugangsleitung zur Folge. Dies kann beispielsweise durch eine entsprechende Ansteuerung des Proportionalventils 307 ausgelöst werden. Die Steuervorrichtung des Simulationssystems steuert dementsprechend das Proportionalventil 307 an, den Durchfluss in die Fluidleitung 324 zu verringern. Da die Blutpumpe des Dialysegeräts 301, die i.d.R. als Schlauchrollenpumpe ausgeführt ist, mit unveränderter Pumpgeschwindigkeit weiter pumpt und bauartbedingt der Fluss konstant bleibt, sinkt infolge des durch das Proportionalventil 307 erhöhten Flusswiderstands stromaufwärts der Blutpumpe des Dialysegeräts der Flüssigkeitsdruck dort. Die Sicherheitsvorrichtung des Dialysegeräts 301 stellt diesen abgesunkenen Flüssigkeitsdruck fest und gibt eine entsprechende Warnmeldung aus. In einem Dialysegerät, in dem die Blutpumpe beispielsweise als Zentrifugalpumpe ausgeführt ist, sinken aufgrund des bauartbedingten Charakters der Zentrifigualpumpe als Konstantdifferenzdruckquelle auch der Fluss und der Flüssigkeitsdruck stromabwärts der Zentrifugalpumpe. Die Sicherheitsvorrichtung eines solchermaßen ausgeführten Dialysegerät 301 würde auch das feststellen und entsprechende Meldungen ausgeben, bzw. Aktionen initiieren, wie das Überführen des Dialysegeräts in einen sicheren Betriebsmodus.

Ein weitere kritische Situation, die während einer Dialysebehandlung auftreten kann betrifft das Abknicken einer Zugangs- oder Rückgabeleitung (116, 117), welches auch Kinking genannt wird.

Ein derartiges Abknicken einer Zugangsleitung kann beispielsweise durch Aktivieren der Abquetschvorrichtungen 330 und/oder 331 simuliert werden. Ein Aktivieren der Abquetschvorrichtung 330 hat eine Erhöhung des Flüssigkeitsdrucks flussabwärts der Blutpumpe des Dialysegeräts 301 zur Folge. Ein Aktivieren der Abquetschvorrichtung 331 hat ein schlagartiges Absenken des Flüssigkeitsdrucks flussaufwärts der Blutpumpe des Dialysegeräts 301 zur Folge. Beides kann von der Sicherheitsvorrichtung des Dialysegeräts detektiert werden und entsprechende Meldungen bzw. Aktionen nach sich ziehen.

Die Folgen des Abknickens einer Zugangsleitung sind teilweise identisch sind mit den Folgen anderer Fehler. Beispielsweise hat ein Ansaugen der arteriellen Nadel die gleichen Folgen wie das Abknicken der arteriellen Zugangsleitung 117. In einer realen Behandlungssituation kann das medizinische Personal überprüfen, ob die Zugangsleitung abgeknickt ist. Wird dieser Zustand in der simulierten Behandlung künstlich verursacht, kann es vorkommen, dass das medizinische Personal die Ursache optisch nicht erkennen kann, insbesondere dann, wenn die Vorrichtungen, die hierzu verwendet werden (bspw. Abquetschvorrichtungen 330, 331) nicht einsehbar sind. Sie können sich beispielsweise uneinsehbar in einem Gehäuse (107) befinden, oder aber der Aktivierungsvorgang ist schlicht durch die Bauart der Vorrichtungen optisch nicht erkennbar.

Für diesen Fall, kann es vorgesehen sein, dass an solchen Vorrichtungen zur Beeinflussung der simulierten Behandlung, deren Aktivierung bzw. Betriebszustand sonst nicht wahrnehmbar ist, Signalisiervorrichtungen, beispielsweise LEDs angebracht sind, die den Betriebszustand der Vorrichtung anzeigen.

Wird beispielsweise ein Abknicken der arteriellen Zugangsleitung 117 simuliert, wird die Abquetschvorrichtung 331 aktiviert und eine ihr zugeordnete Signalisiervorrichtung, beispielsweise eine LED zum Leuchten gebracht.

Ebenso kann ein Herausrutschen einer Nadel, wenn es in der Simulation nicht tatsächlich passiert, sondern die Folgen durch das entsprechende Ansteuern von Vorrichtungen (Ansteuern von des 3-Wegeventils 306) bewirkt werden, durch eine Signalisiervorrichtung an der Punktionsstelle, beispielsweise eine Kette roter LEDs, die eine Blutspur symbolisiert, angezeigt werden.

Vorstellbar ist auch, dass nicht nur die Folgen von realen Störungen bei der Simulation bewirkt werden, sondern die Störung selbst. D.h. es kann vorgesehen sein, dass beispielsweise die Nadeln, die die externen Fluidleitungen 116 und 117 mit den internen, im künstlichen Patientenarm befindlichen Fluidleitungen 324 und 325 verbinden, durch entsprechende Aktoren verfahrbar sind. So kann tatsächlich ein Herausrutschen einer Nadel erwirkt werden, indem ein Aktor, beispielsweise ein elektrisch ansteuerbarer Hubmagnet, die Nadel aus dem Patientenarm bewegt.

Austretende Blutersatzflüssigkeit kann in einem solchen Fall auch durch dem Fachmann bekannte Feuchtedetektoren detektiert werden und am Dialysegerät als Alarmmeldung angezeigt werden. Derartige Sensoren werden auch in der realen Behandlung zur Detektion von austretendem Blut benutzt. Ein derartiger Feuchtedetektor ist beispielsweise in der WO08021462 A2 beschrieben. Die Funktionsweise eines solchen Feuchtedetektors basiert beispielsweise darauf, dass austretende Flüssigkeit den elektrischen Widerstand zwischen elektrischen Kontakten verändert.

Es ist vorstellbar, dass bei der Verwendung eines Feuchtesensors bei der Simulation der Behandlung dieser derart ausgeführt ist, dass der Widerstand zwischen den elektrischen Kontakten aktiv steuerbar ist, beispielsweise indem ein ansteuerbarer Schalter (Transistor, Relais) vorgesehen ist, der ursprünglich voneinander isolierte Leiterbahnen leitend miteinander verbindet. In dieser Ausführungsform kann der Sensor auch ohne austretende Flüssigkeit seinen Widerstand ändern, was von der Sicherheitsvorrichtung des mit diesem Sensor ausgerüsteten Dialysegeräts als Blutaustritt interpretiert werden würde. Im Zusammenspiel mit den weiter oben beschriebenen Maßnahmen zur Simulation eines Nadelverlustes kann die Realitätsnähe so weiter gesteigert werden.

Auch ein Ansaugen der Nadel an einer Gefäßwand kann erwirkt werden, in dem die Nadel entsprechend so bewegt wird, dass sie sich tatsächlich an eine künstliche Gefäßwand ansaugt.

Es ist aber auch vorstellbar, dass das Dialysegerät selbst von der Steuervorrichtung des Simulationsystems zur Ausgabe von Warnungen angesteuert wird. In diesem Fall ist das Dialysegerät derart ausgeführt, dass das Simulationssystem Zugriff auf die Steuerung des Dialysegeräts hat.

Die Pumpe 303 ist in einer Ausführungsform eine Zentrifugalpumpe. Zentrifugalpumpen (auch als Impellerpumpen bezeichnet) zeichnen sich durch eine bauartbedingte Druckbegrenzung aus. Dies hat den Vorteil, dass im Fluidsystem nach Figur 3 kein gefährlicher Überdruck aufgebaut werden kann, unabhängig von der Stellung der einzelnen Ventile oder Durchflussdrosseln.

Das in Figur 3 dargestellte Fluidsystem ermöglicht es, die Flüssigkeitsdrücke und die Flüsse in den einzelnen Zweigen im künstlichen Patientenarm 203 unabhängig voneinander einzustellen, beispielsweise in dem die Proportionalventile 307 und 308 (Drosselventile) entsprechend angesteuert werden.

Sämtliche Aktoren (Pumpen, Ventile) des Fluidsystems nach Figur 3 sind in einer Ausführungsform geräuscharm ausgeführt, um dem medizinischen Personal, das mit der simulierten Behandlung befasst ist, keine anderen Hinweise auf Störungen zu geben, als solche, die auch in der Realität auftreten würden.

In einer Ausführungsform kann es vorgesehen sein, dass die Viskosität der Blutersatzflüssigkeit beeinflussbar ist. Hierzu kann als Blutersatzflüssigkeit eine magnetorheologische und/oder elektrorheologische Flüssigkeit verwendet werden. Eine magnetorheologische Flüssigkeit enthält magnetisch polarisierbare Partikel, beispielsweise Carbonyleisenpulver, die sich durch ein angelegtes magnetisches Feld polarisieren und innerhalb der Flüssigkeit, in der sie gelöst sind, neu orientieren lassen. Hierdurch kann die Viskosität der Flüssigkeit aktiv verändert werden. Elektrorheologische Flüssigkeiten lassen sich hingegen durch das Anlegen eines elektrischen Felds in ihrer Viskosität beeinflussen.

Eine weitere Möglichkeit, die Viskosität der Blutersatzflüssigkeit zu beeinflussen, besteht in der Zumischung von die Viskosität beeinflussenden Stoffen, beispielsweise Hydroxyethylstärke, abgekürzt HES oder Polyethylenglycol, kurz PEG. Diese können in Behältnissen vorgehalten werden und bedarfsweise der Blutersatzflüssigkeit zugemischt werden, um deren Viskosität zu erhöhen. Eine Erniedrigung der Viskosität erfolgt beispielsweise durch Zusetzen von Wasser.

Mit der aktiven Beeinflussung der Viskosität der Blutersatzflüssigkeit lässt sich beispielsweise die Erhöhung der Viskosität des Blutes durch Abfiltrieren von Wasser über die Dialysemembran (Ultrafiltration) simulieren. Dialysegeräte können mit entsprechenden Viskositätssensoren ausgerüstet sein, die eine derartige Veränderung der Viskosität der Blutersatzflüssigkeit detektieren können.

In einer weiteren Ausführungsform ist die Temperatur der Blutersatzflüssigkeit einstellbar. Hierzu können dem Fachmann bekannte Heiz-/Kühlvorrichtungen vorgesehen sein, die eine auswählbare Temperatur der Blutersatzflüssigkeit herbeiführen. Über die Temperatur der Blutersatzflüssigkeit lassen sich weitere medizinische Situationen simulieren, beispielsweise Fieber oder Frieren des Patienten. Dialysegeräte sind oftmals mit Sensoren zur Temperaturerfassung im extrakorporalen Blutkreislauf ausgerüstet. Somit kann bei einer Abweichung der Bluttemperatur von einem Erwartungswert eine Alarmmeldung ausgegeben werden, auf die die Testperson reagieren muss, beispielsweise mit manueller Bestimmung der Körpertemperatur und Ergreifen von Gegenmaßnahmen.

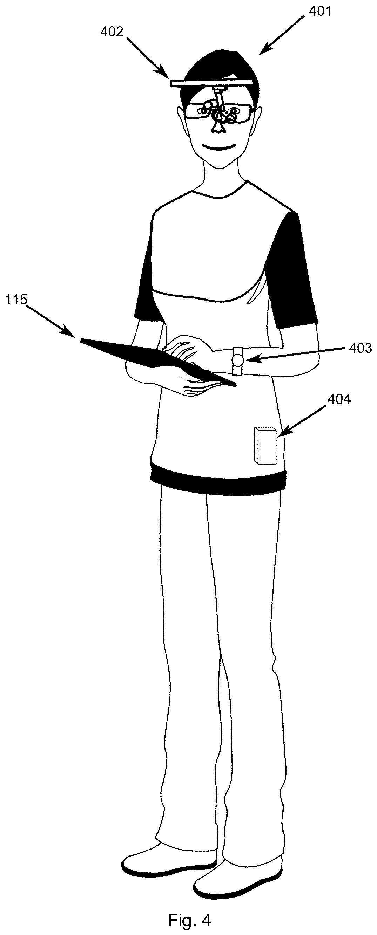

Die Figur 4 zeigt eine an dem Simulationssystem 100 trainierende Testperson 401 (analog der Testperson 106 aus Figur 1) mit mehreren Überwachungsvorrichtungen 402, 403, 404, die dazu dienen, das Verhalten und physiologische Parameter der trainierenden Person 401 zu überwachen.

So kann über eine sogenannte Eye-Tracking Brille 402 detektiert werden, auf welchen Punkt die Testperson blickt. Hierzu ist die Brille 402 mit Kameras ausgestattet, die einerseits auf die Augen der Testperson und andererseits in Blickrichtung gerichtet sind. Über eine spezielle Auswertelogik kann aus den beiden Kamerabildern errechnet werden, auf welchen Punkt die Testperson 401 blickt. Eye-Tracking Vorrichtungen kennt der Fachmann beispielsweise aus der WO2004066097A2.

Durch die Überwachung der Blickrichtung, sowie der zugehörigen Punkte, auf die die Testperson 401 blickt (Fokussierpunkte), können Rückschlüsse auf den Trainingszustand der Testperson gewonnen werden. So kann beispielsweise die Schnelligkeit, mit der bestimmte Bedienpunkte an dem Dialysegerät anvisiert werden, beispielsweise in Reaktion auf bestimmte simulierte medizinische Vorkommnisse, ein Maß dafür sein, wie gut die Testperson das an der Simulation beteiligte Dialysegerät kennt. Es kann auch bewertet werden, wie sicher und schnell die Testperson notwendige Handlungen am künstlichen Ersatzpatienten ausführt. Eine Handlung am künstlichen Ersatzpatienten kann als Reaktion auf simulierte medizinische Ereignisse erforderlich sein, beispielsweise kann in schon beschriebener Weise ein Nadelverlust simuliert werden, wobei bewertet wird, wie schnell und zielgerichtet, also ohne längeres Suchen, der Blick der Testperson auf die potentielle Fehlerquelle, in diesem Fall die Punktionsstelle, gerichtet wird.

Die Überwachung der Blickrichtung der Testperson 401 kann aber auch dazu dienen, Informationen über die Bedienbarkeit eines medizinischen Geräts zu gewinnen. So kann beispielsweise bewertet werden, ob ein bestimmtes Bedienkonzept zu einer schnellen und sicheren Bedienung führt, indem die Blickrichtungen bei der Bedienung ausgewertet werden. Eine derartige Bewertung, beispielsweise im Rahmen einer Risikoanalyse, kann auch medizinische Verfahren betreffen, beispielsweise bestimmte Vorschriften zur Aufrüstung des medizinischen Geräts oder beim Umgang mit dem Patienten.

Generell dient die Überwachung von physiologischen Parametern der Testperson 401 der Bewertung ihrer physischen und psychischen Belastung. Alle Sensoren, die hierfür relevante Parameter erfassen, können hierfür verwendet werden. In der Figur 4 beispielhaft gezeigt ist beispielsweise ein Gerät 403 zum Messen des Blutdrucks, des Pulses, der Hautfeuchtigkeit bzw. des Hautwiderstands usw., welche als Indikatoren zur physischen und psychischen Belastung herangezogen werden können. Das Gerät 403 hat in Figur 4 beispielhaft die Form eines Armbands.

Das Gerät 404 symbolisiert eine Vorrichtung zur Erfassung beliebiger physiologischer Parameter, wie beispielsweise Körpertemperatur, EKK, EEG, Atemfrequenz usw. Die dazu notwendigen Ausführungen sind dem Fachmann aus dem Stand der Technik bekannt. Die so erfassten physiologischen Parameter werden in Bezug auf die simulierte Behandlung gesetzt und von Experten bewertet, um Aussagen über die Testperson und/oder die Simulationsumgebung (Bedienbarkeit, medizinische Verfahren) treffen zu können. Die erfassten physiologischer Parameter können hierzu mit der Audio- und Videoaufzeichnung kombiniert werden, die entsprechend in die Bewertung mit einfließt.

Durch die so durchgeführte Bewertung des Trainingszustands der Testperson ist es möglich, den Qualifikationsgrad der Testperson objektiv zu messen und zu zertifizieren. So können Simulationen mit Aufgaben in unterschiedlichen Schwierigkeitsgraden durchgeführt werden, die die Testperson bewältigen soll. Die Anforderungen an medizinisches Hilfspersonal sind i.d.R. geringer als die an reguläres medizinisches Personal, wobei hier auch unterschieden werden kann beispielsweise in "qualifiziert zur Betreuung normaler Behandlungen", oder "qualifiziert zur Betreuung akuter Behandlungen", oder "qualifiziert zur Betreuung von Intensivbehandlungen". Je nach Qualifikationsgrad unterscheiden sich die in der Simulation gestellten Aufgaben. D.h. die medizinischen bzw. technischen Situationen in der Behandlungssimulation unterscheiden sich je nach zu zertifizierendem Qualifikationsgrad von beispielsweise "einfach" für medizinisches Hilfspersonal, über "normal" für reguläres medizinisches Personal bis hin zu "schwierig" für medizinisches Personal in Intensivstationen oder Ärzte.

Durch wiederkehrendes Ablegen von derartigen Qualifikationszertifizierungen wird garantiert, dass das medizinische Personal sich fortwährend weiterbildet und dementsprechend die Qualität und Sicherheit der Behandlung gewährleistet.

Zur Zertifizierung können beliebige während der Simulation aufgezeichnete Parameter herangezogen werden. Beispielweise kann die Anzahl der richtig gelösten Aufgaben während einer Behandlungssimulation auf die hierfür gebrauchte Zeit bezogen werden. Alternativ können auch die gemessenen physiologischen Parameter der Testperson in eine derartige Zertifizierung mit einfließen.

Die Testperson 401 kann einen mobilen Computer 115 (Tablet-PC oder Smartphone) mit sich führen. Der mobile Computer 115 kann eine Software geladen haben und ausführen, die die Simulation auf vielfältige Weise unterstützt. So kann zur Evaluierung des Lerneffekts auf dem mobilen Computer 115 bei einer simulierten medizinischen Situation, beispielsweise eine Alarmsituation, der Testperson 401 eine Auswahlmöglichkeit an möglichen Ursachen für die aufgetretene medizinische Situation angezeigt werden. Die Testperson kann hiervon eine ihr richtig erscheinende Ursache auswählen. Die Auswahl kann dokumentiert und bewertet werden, wobei die Bewertung einschließt, ob die Auswahl korrekt war und wie schnell die Testperson die Auswahl getroffen hat. Diese Faktoren können in die Zertifizierung der Testperson mit einfließen.

Darüber hinaus ist es möglich, dass der mobile Computer 115 eine Online Hilfe anbietet. Hat die Testperson beispielsweise Schwierigkeiten, auf eine simulierte medizinische Situation korrekt zu reagieren, kann eine Online Hilfe angeboten werden, die konkrete Hintergrundinformationen zur aufgetretenen medizinischen Situation anbietet. Eine derartige Online Hilfe kann Zugriff auf externe Datenbanken haben.

Weiterhin kann vorgesehen sein, dass die Testperson über den mobilen Computer 115 mit einem Experten direkt kommuniziert. Hierzu kann eine Audio und/oder Videoübertragung vorgesehen sein, die zwischen Testperson und Experten stattfindet. Es ist somit möglich, dass ein Experte mit der Testperson direkt kommuniziert, um Fragen zu beantworten, Hinweise oder Anweisungen zu geben.

Der mobile Computer 115 ist hierzu mit einer entsprechenden Software programmiert, die mit der Steuerungssoftware der restlichen Simulationsumgebung interagieren kann. Hierzu können datentechnische Verbindungen zwischen dem mobilen Computer 115 und den Steuer- und Aufzeichnungsgeräten 104a und 104b, sowie zu sonstigen Netzwerken, beispielsweise dem Internet vorgesehen sein. Diese datentechnischen Verbindungen sind vorteilhaft drahtlos. Zur Kommunikation zwischen Testperson und Experten können die oftmals standardmäßig vorhandene Kamera, Mikrofon und Lautsprecher des mobilen Computers benutzt werden.

In einer Ausführungsform können die beschriebenen vom mobilen Computer ausgeführten Verfahren auch vom medizintechnischen Gerät ausgeführt werden. Hierzu kann eine als Touchscreen Display ausgeführte Bedienerschnittstelle des medizintechnischen Geräts als Ein/Ausgabevorrichtung analog der Ausführung mit dem mobilen Computer verwendet werden. Ebenfalls denkbar ist, dass das medizintechnische Gerät mit einer Kamera, einem Mikrofon und einem Lautsprecher ausgestattet ist, über die die Testperson mit Experten in schon beschriebener Weise kommuniziert. Das medizinische Gerät ist in dieser Ausführungsform also dazu eingerichtet, eine audiovisuelle Kommunikation mit einem entfernten Gerät aufzubauen. Das medizintechnische Gerät ist hierzu mit einer entsprechenden Software programmiert, die mit der Steuerungssoftware der restlichen Simulationsumgebung interagieren kann.

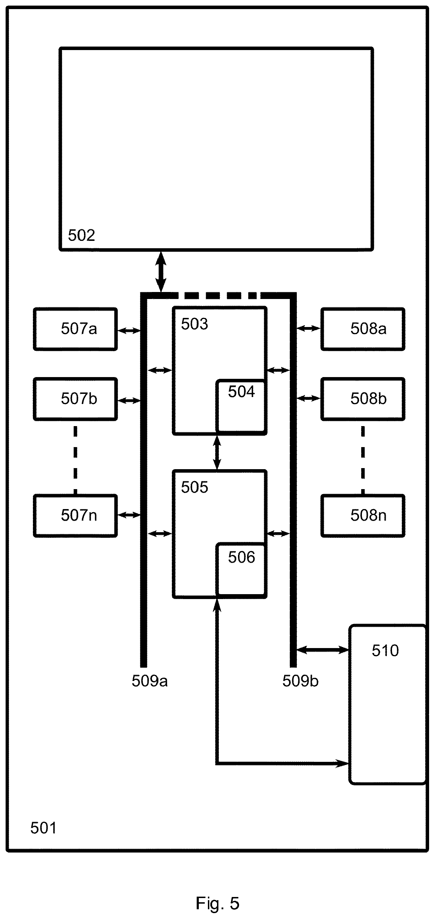

Die Figur 5 zeigt in einer schematischen Darstellung ein medizintechnisches Gerät 501 (analog dem als Hämodialysegerät ausgeführtem medizintechnischem Gerät 101 aus Figur 1) mit wesentlichen Komponenten.

Das medizintechnische Gerät 501 ist i.d.R. mit einer Bedienerschnittstelle 502 ausgestattet, die vorteilhaft als Touchscreen Display, also ein Bildschirm mit berührempfindlicher Oberfläche zur Entgegennahme von Bedienereingaben, ausgeführt ist.

Medizintechnische Geräte sind i.d.R. von einem oder mehreren Steuergeräten gesteuert, welche vorteilhaft softwareprogrammiert werden können. Im Lichte der Offenbarung der vorliegenden Erfindung ist durch die Nennung von einem Steuergerät (Einzahl) auch immer eine Vielzahl von Steuergeräten mit umfasst. Die hierzu notwendige Software kann in einem Datenspeicher abgelegt sein, der darüber hinaus auch zur Datenspeicherung sonstiger Daten verwendet werden kann

In der Figur 5 ist das medizintechnische Gerät 501 exemplarisch mit zwei Steuergeräten 503 und 505 mit jeweilig separatem Datenspeichern 504 und 506 ausgestattet. Hierbei kann das Steuergerät 503 als zentrales Steuergerät zur Steuerung des Betriebs des medizintechnischen Gerätes dienen, während das Steuergerät 505 als Sicherheits- und Überwachungssteuergerät unabhängig vom Zustand des Steuergeräts 503 agieren kann.

Weiterhin kann das medizintechnische Gerät 501 mit zumindest einem Aktor 507a bis 507n und zumindest einem Sensor 508a bis 508n ausgerüstet sein. Aktoren sind insbesondere elektromechanische Wandler wie Pumpen, Ventile, Motoren, Stellglieder, Lautsprecher etc., aber auch Wärme- oder Kälteerzeuger oder auch Hydraulik- oder Pneumatikelemente. Aktoren und Sensoren wirken zusammen, um die mit dem medizintechnischen Gerät 501 mögliche Behandlung durchzuführen und werden hierbei von den Steuergeräten 503 bzw. 503 und 505 gesteuert und überwacht.

Hierzu können die Aktoren 507a bis 507n und die Sensoren 508a bis 508n datentechnisch mit dem Steuergerät 503 bzw. mit den Steuergeräten 503 und 505 verbunden sein. In der Figur 5 ist ein Datenaustausch zwischen zwei Komponenten durch einen Doppelpfeil symbolisiert. Zum Datenaustauch verfügt das medizintechnische Gerät 501 in Figur 5 exemplarisch über zumindest einen Datenbus 509a und 509b. Durch die gestrichelte Linie in Figur 5, die den Datenbus 509a und 509b verbindet, ist die Ausführungsform mit nur einem Datenbus angedeutet. Die Datenbusse 509a und 509b können auch getrennt und unabhängig voneinander vorliegen. Die Datenbusse können insbesondere nach dem Industriestandard CAN ausgeführt sein.

Das medizintechnische Gerät 501 verfügt zum Datenaustausch mit externen Geräten oder Netzwerken über eine Datenschnittstelle 510. Diese kann beliebig ausgeführt sein, beispielsweise als Kabelverbindung oder drahtlos. Die Datenübertragung kann nach einem beliebigen Datenübertragungsprotokoll stattfinden, insbesondere können gängige Internetprotokolle wie Transmission Control Protocol/Internet Protocol (TCP/IP) verwendet werden. Es können aber auch proprietäre Datenübertragungsprotokolle verwendet werden.

Über die Datenschnittstelle kann Einfluss auf das Steuergerät des medizintechnischen Geräts 501 genommen werden. So kann das medizintechnische Gerät 501 zum Zwecke der Verwendung in dem der Erfindung zugrunde liegendem Simulationssystem derart konfiguriert sein, dass weitere Steuergeräte, wie beispielsweise die Steuer- und Aufzeichnungsgeräte 104a und 104b über die Datenschnittstelle mit dem Steuergerät des medizintechnischen Geräts zusammenwirken, um das medizintechnische Gerät entsprechend der zu simulierenden medizinischen Behandlung anzusteuern. Um eine konkrete medizinische Situation zu simulieren, kann das medizintechnische Gerät 501 entweder durch Ansteuern von Aktoren in den entsprechenden Zustand gebracht werden. Es können aber auch andererseits Sensorwerte, also die Ausgangsparameter der Sensoren 508a bis 508n, derart durch die Steuerung durch beispielsweise das externe Steuer- und Aufzeichnungsgerät (104a,104b) verändert bzw. manipuliert werden, dass dem Steuergerät (503, 505) des medizintechnischen Geräts bestimmte Werte übermittelt werden, die nicht den realen Werten entsprechen, aber z.B. charakteristisch für bestimmte Alarmbedingungen sind. Das Steuergerät des medizintechnischen Geräts stellt entsprechend seiner Programmierung in Folge dieser manipulierten Sensorwerte eine Alarmsituation fest und gibt eine entsprechende Alarmmeldung vorzugsweise auf der Bedienerschnittstelle 502 aus. Eine am Simulationssystem teilnehmende Testperson kann auf diese angezeigte Alarmmeldung reagieren.

Beispielsweise könnte bei einem als Hämodialysegerät ausgeführten medizintechnischen Gerät, mit dem eine Dialysebehandlung simuliert werden soll, der Sensorwert des venösen Drucksensors, also des Sensors, der den Blutdruck im venösen Blutschlauch misst, derart manipuliert werden, dass das Steuergerät des Hämodialysegeräts auf den Verlust der venösen Nadel schließt, was eine potentiell gesundheitsgefährdende Situation für einen angeschlossenen Patienten darstellt. In Folge dieses manipulierten Sensorwertes kann das Dialysegerät die Behandlung unterbrechen und sich in einen sicheren Betriebszustand begeben (beispielsweise Stopp der Blutpumpe, Abklemmen der Patientenblutschläuche). Ein dem manipulierten Sensorwert entsprechende Alarmmeldung, beispielsweise "venöser Druckalarm" kann vom Hämodialysegerät ausgegeben werden.

Es kann vorgesehen sein, dass das externe Steuer- und Aufzeichnungsgerät (104a,104b) zeitgleich auch eine Signalisiervorrichtung an der Punktionsstelle des künstlichen Ersatzpatienten 105, beispielsweise eine Kette roter LEDs, die eine Blutspur symbolisiert, aktiviert.

Es kann vorgesehen sein, dass die an der Simulation teilnehmende Testperson die von ihm diagnostizierte Ursache der Alarmmeldung in den mobilen Computer 115 eingibt, der seinerseits in Datenverbindung mit dem externen Steuer- und Aufzeichnungsgerät (104a,104b) steht und so die Eingabe der Testperson übermitteln kann. Anstelle des mobilen Computers 115, kann auch das medizintechnische Gerät 501 in schon beschriebener Weise programmiert sein, und die gleichen Aufgaben wie der mobile Computer 115 zu erfüllen.

Das externe Steuer- und Aufzeichnungsgerät kann derart programmiert sein, dass die Eingabe der Testperson verifiziert wird. Abhängig von der Eingabe der Testperson am mobilen Computer 115 (oder dem medizintechnischen Gerät 501) und/oder von Handlungen an dem Hämodialysegerät, die ebenfalls über eine Datenverbindung vom Hämodialysegerät an das externe Steuer- und Aufzeichnungsgerät gemeldet werden können, können weitere Steuerbefehle an das Hämodialysegerät, den künstlichen Patienten 105 (und verbundenen Vorrichtungen) und/oder den mobilen Computer 115 gesendet werden.

So kann bei Eingabe der korrekten Ursache für einen angezeigten Alarm das externe Steuergerät veranlassen, dass wieder der reale arterielle Druckwert dem internen Steuergerät des Hämodialysegeräts übermittelt wird. I.d.R. muss die Testperson die Beseitigung der Alarmursache auch an der Bedienerschnittstelle des Hämodialysegeräts bestätigen, woraufhin es mit der Behandlung fortfahren kann.

Es kann auch vorgesehen sein, dass das medizintechnische Gerät 501 zusätzliche Vorrichtungen, insbesondere Aktoren aufweist, um das medizintechnische Gerät gezielt zu beeinflussen. Beispielsweise können bei einem als Hämodialysegerät ausgeführten medizintechnischen Gerät die Blutschläuche durch eine ansteuerbare Klemme (elektromechanisch, pneumatisch oder hydraulisch) real verschlossen werden, um Kinking, also das Abquetschen eines Schlauches, zu simulieren, oder über eine zusätzliche Pumpe Luft in den extrakorporalen Blutkreislauf eingebracht werden, um eine Undichtigkeit zu simulieren.

Das an dem Simulationssystem beteiligte medizintechnische Gerät kann derart ausgeführt sein, dass bestimmte Betriebszustände, wie beispielsweise Alarmbedingungen, real herbeigeführt werden, oder aber nur die Steuerung des medizintechnischen Geräts derart manipuliert wird, dass es auf eine Alarmbedingung schließt, obwohl sie real nicht vorliegt.

Unabhängig von der Ausführung des medizintechnischen Geräts kann es derart ausgeführt sein, dass das externe Steuer- und Aufzeichnungsgerät (104a, 104b) Zugriff auf seine grundlegenden Steuerfunktionen hat. Dies kann eine reine Software basierte Ausführung umfassen, es kann aber auch zusätzliche Hardware notwendig sein, die den Datenverkehr zwischen dem internen Steuergerät (503, 505) des medizintechnischen Geräts 501 und den Aktoren (507a-n) und Sensoren (508a-n) manipulieren kann.

Im Folgenden werden einige Betriebszustände eines als Hämodialysegerät ausgeführten medizintechnischen Geräts, die vom externen Steuer- und Aufzeichnungsgerät (104a,104b) erwirkt werden können, beschrieben:
Über den Zugriff auf beliebige Variablen in der Software des Hämodialysegeräts ist es möglich, die Ausgangsvariablen beliebiger Sensoren des Gerätes so zu manipulieren, dass falsche Werte weitergegeben werden, so dass in der Folge alle möglichen Alarme erzeugt werden können. Derartige Alarme können Druckalarme im extrakorporalen Blutkreislauf und im Dialysatkreislauf betreffen. Mögliche andere Alarme sind Blutleckalarme, beispielsweise bei Undichtigkeiten im extrakorporalen Blutkreislauf oder bei der Ruptur der Dialysatormembran, bei der Blut aus dem Blutkreislauf in den Dialysatkreislauf übertritt und dort von einem Blutlecksensor detektiert werden kann. Weiterhin kann Lufteintritt in den extrakorporalen Blutkreislauf einen entsprechenden Alarm auslösen. Weiterhin können sämtliche Aktoren, beispielsweise die Blutpumpe, manipuliert werden, um beispielsweise einen Ausfall des Aktors zu erwirken. Möglich ist es auch, einen kompletten Ausfall der Stromversorgung zu simulieren, beispielsweise durch Ansteuern eines Relais, welches das Hämodialysegerät von der Stromversorgung trennt.

Undichtigkeiten im extrakorporalen Blutkreislauf können auch durch Flüssigkeit in einem Auffangbeutel simuliert werden, der bei einigen Hämodialysegeräten zum Auffangen austretender Flüssigkeiten vorgehalten wird. Austretende Flüssigkeit kann aber auch zunächst in einer Auffangrinne gesammelt und einem Feuchtigkeitssensor zugeführt werden. Zur Ausbringung von Flüssigkeit kann eine dezidierte Pumpe vorgesehen sein, es kann aber auch ein Ventil vorgesehen sein, dass steuerbar Flüssigkeit aus dem extrakorporalen Blutkreislauf abzweigt.

Um beispielsweise eine Leckage eines Blutschlauches zu simulieren, kann beispielsweise ein solcher Feuchtigkeitssensor, der zum Detektieren austretender Flüssigkeit vorgehalten wird, manipuliert werden. Es kann aber auch über eine zusätzliche Pumpe oder ein Abzweigventil reale Flüssigkeit an den Feuchtigkeitssensor geführt werden. Es kann vorgesehen sein, dass die Testperson die Flüssigkeit entfernen muss, bevor die Behandlung fortgesetzt werden kann, was einer realen Behandlung weitgehend entspricht.

Mögliche Fehler, die bei der Behandlung vorkommen und auf die beschriebene Weise simuliert werden können, sind weiterhin eine versehentlich nicht geöffnete Schlauchklemme oder die Simulation eines durch Blutgerinnung verstopften Dialysators (Clotting), das letztere zu erkennen am Transmembrandruckwert, also der Differenz des (mittleren) Flüssigkeitsdruckes auf der Blutseite und der Dialysatseite der Dialysatormembran.

Bei der Simulation verschiedener Vorkommnisse kann abgewogen werden, auf welche Weise simuliert werden soll. So ist es vorteilhaft, Luft im extrakorporalen Blutkreislauf real mit echter Luft zu simulieren, also beispielsweise durch eine entsprechende Pumpe Luft in den extrakorporalen Blutkreislauf zu pumpen. Wird der Luftalarm nur durch die Manipulation interner Gerätevariablen simuliert, besteht die Möglichkeit, dass eine Testperson den Luftalarm als Fehlalarm interpretiert, weil keine Luft im extrakorporalen Blutkreislauf zu finden ist.

Die durch die weiteren Steuergeräte, wie beispielsweise die Steuer- und Aufzeichnungsgeräte 104a und 104b, herbeiführbaren Betriebszustände entsprechen sinngemäß abnormalen Betriebszuständen, da sie Fehler oder Alarmzustände herbeiführen oder simulieren.

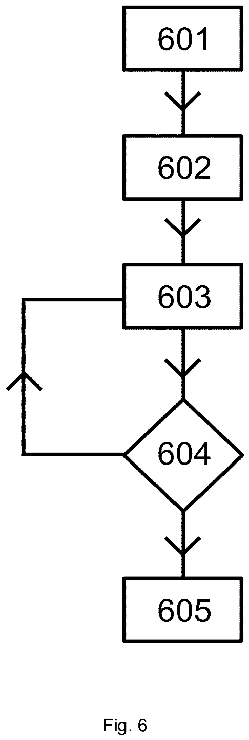

Die Figur 6 zeigt ein schematisches Ablaufdiagramm für ein Verfahren im Einklang mit der Lehre der vorliegenden Erfindung.

Das Verfahren startet im Schritt 601. Im Schritt 602 wird mit der Aufzeichnung der Simulation begonnen. Diese Aufzeichnung kann die Aufzeichnung aller möglichen Parameter der Trainingsperson beispielsweise durch die Überwachungsvorrichtungen 402, 403, 404 in Figur 4, die dazu dienen, das Verhalten und physiologische Parameter der trainierenden Person 401 zu überwachen, umfassen. Weiterhin kann die Aufzeichnung der Simulation auch die Audio und Videoaufzeichnung (beispielsweise mit der Kamera 109 in Figur 1) umfassen. Weiterhin kann die Aufzeichnung der Simulation auch umfassen, alle Betriebsparameter der beteiligten Vorrichtungen, insbesondere der an der Simulation beteiligten (medizintechnischen) Geräte und des künstlichen Patienten aufzuzeichnen. Die Betriebsparameter einer Vorrichtung kennzeichnen im weitesten Sinn den aktuellen Zustand der Vorrichtung. Diese können Zustandsparameter von Aktoren und Sensoren der Vorrichtung, Variablenwerte der Softwaresteuerung, Strom- und Spannungsverläufe der elektrischen Versorgung etc. umfassen und sind mit einem eindeutigen Zeitpunkt verknüpft.

Weiterhin kann die Aufzeichnung auch umfassen, dass alle Eingaben, die die Testperson in den mobilen Computer 115 oder ein entsprechen programmiertes medizintechnisches Gerät und auch die Ausgaben, insbesondere die Displayausgabe des mobilen Computers 115 oder des medizintechnischen Geräts abgespeichert werden.

Die Aufzeichnung der Simulation im Schritt 602 erfolgt durch entsprechende Vorrichtungen, wie beispielsweise durch die Steuer- und Aufzeichnungsgeräte 104a und 104b in Figur 1, die als Computer ausgeführt sein können. Die anfallenden Daten können lokal in den Steuer- und Aufzeichnungsgeräten 104a und 104b gespeichert werden und/oder in einem entfernten Speichermedium, beispielsweise in einem durch ein Datennetzwerk verbundenen Server, oder in einer Datenwolke (Cloud).

Sinn der Aufzeichnung der Simulation ist es, die Aktionen und den Zustand der Testperson während der Simulation mit den Abläufen und den Zuständen der an der Simulation beteiligten Geräte und des künstlichen Patienten zu verknüpfen, um so Erkenntnisse hinsichtlich des Qualifikationsgrads der Testperson und/oder der Bedienbarkeit der an der Simulation beteiligten Geräte ableiten zu können.

Die Aufzeichnung wird in einer Ausführungsform mindestens bis zur Beendigung des Verfahrens im Schritt 605 durchgeführt.

Im Schritt 603 erfolgt die Initiierung einer Zustandsänderung der Simulationsumgebung. Eine Zustandsänderung der Simulationsumgebung dient dazu, ein bestimmtes Szenario, welches bei einer realen Behandlung vorkommen kann, zu simulieren. Hierzu können in schon beschriebener Weise alle an der Simulation beteiligten Vorrichtungen, insbesondere das medizintechnische Gerät und der künstliche Ersatzpatient derart angesteuert werden, dass sie einen bestimmten Zustand annehmen. Denkbar ist auch, dass die Umweltvariablen im Simulationsraum 102 beeinflusst werden, beispielsweise die Beleuchtung, die Temperatur, die Belüftung oder die Luftfeuchte im Simulationsraum 102 verändert oder Geräusche über Lautsprecher eingespielt werden.

Im Schritt 604 wird ein Abbruchkriterium der Simulation überprüft. Ein solches Abbruchkriterium kann eine Bedienereingabe beispielsweise des Beobachters 110 sein, der das Verfahren hierdurch beendet. Andere Abbruchkriterien können auch ein abgelaufener Timer sein, wodurch eine festgelegte Simulationszeit realisiert wird, oder das Eintreten einer schädlichen Situation während der Simulation, die ein sofortiges Abbrechen der Simulation notwendig macht.

Trifft das Abbruchkriterium ein, erfolgt Schritt 605, nämlich das Beenden der Simulation. Trifft das Abbruchkriterium nicht ein, wird der Schritt 603 erneut ausgeführt.

Das Beenden der Simulation kann umfassen, dass die an der Simulation beteiligten Vorrichtungen in einen sicheren Zustand versetzt werden.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand oder dem erfindungsgemäßen Verfahren vorgesehen sein, um gleichzeitig deren vorteilhafte Wirkungen zu realisieren. Es liegt somit z.B. ebenso im Rahmen der Erfindung alternativ oder kumulativ zu den an der Simulation beteiligten Vorrichtungen, andere Vorrichtungen, die z.B. Einfluss auf die simulierte medizinische Behandlung haben, in die Simulationsumgebung zu integrieren, oder Vorrichtungen, die in den konkreten Ausführungsbeispielen als getrennt vorliegende Einzelvorrichtungen beschrieben sind, in einer übergeordneten Vorrichtung zu integrieren. So können beispielsweise die Steuer-und Aufzeichnungsgeräte 104a,b in einer Ausführungsform auch als integraler Bestandteil eines an der Simulation einer medizinischen Behandlung beteiligten medizintechnischen Geräts vorliegen.

Für einen Fachmann ist es insbesondere offensichtlich, dass die Erfindung nicht nur bei Dialysegeräten und damit ausgeführte Behandlungen angewendet werden kann, sondern auch bei beliebigen anderen medizintechnischen Geräten und damit ausführbaren medizinischen Behandlungen.

Der Schutzbereich der vorliegenden Erfindung ist durch die Ansprüche gegeben und wird durch die in der Beschreibung erläuterten oder den Figuren gezeigten Merkmale nicht beschränkt.

Die vorliegende Erfindung betrifft insbesondere folgende Aspekte:
A1. Verfahren zur Simulation medizinischer Behandlungen mit den Schritten:
   Bereitstellen mindestens eines medizintechnischen Geräts als Teil einer Simulationsumgebung, dessen Betriebsparameter steuer-und beobachtbar sind;
   Aufzeichnen von Simulationsdaten, wenigstens für die Dauer der Simulation,
   Initiierung einer Zustandsänderung der Simulationsumgebung,
   Überprüfen eines Abbruchkriteriums.
A2. Verfahren nach Aspekt A1 mit dem zusätzlichen Schritt. Bereitstellen eines künstlichen Ersatzpatienten als Teil der Simulationsumgebung, wobei der künstliche Ersatzpatient mit dem medizintechnischen Gerät zum Zweck einer simulierten Behandlungsausführung interagieren kann und dessen Betriebsparameter steuer- und beobachtbar sind.
A3. Verfahren nach Aspekt A2, wonach die Simulationsdaten Betriebsparameter des künstlichen Ersatzpatienten umfassen.
A4. Verfahren nach einem der vorhergehenden Aspekte, wonach die Simulationsdaten ausgewählt sein können aus der folgenden Gruppe:
   physiologische Daten einer Testperson;
   Fokussierpunkte einer Testperson;
   Audio- und/oder Videoaufzeichnungen einer Testperson;
   Eingaben einer Testperson in eine Bedienerschnittstelle;
   Betriebsparameter des medizintechnischen Geräts.
A5. Verfahren nach einem der vorhergehenden Aspekte, wonach die Initiierung der Zustandsänderung der Simulationsumgebung umfasst:
   Ansteuern des medizintechnischen Geräts und/oder einer mit dem medizintechnischen Gerät interagierenden Vorrichtung derart, dass das medizintechnische Gerät und/oder die mit dem medizintechnischen Gerät interagierende Vorrichtung seinen momentanen Betriebszustand in einen anderen Betriebszustand überführt.
A6. Verfahren nach Aspekt A2, wonach die Initiierung der Zustandsänderung der Simulationsumgebung umfasst:
   Ansteuern des künstlichen Ersatzpatienten derart, dass der künstliche Ersatzpatient seinen momentanen Betriebszustand in einen anderen Betriebszustand überführt.
A7. Verfahren nach einem der vorhergehenden Aspekte, wonach die Initiierung der Zustandsänderung der Simulationsumgebung umfasst:
   Ändern der Umweltvariablen in einem Simulationsraum, wobei die Umweltvariablen des Simulationsraums ausgewählt sind aus der Gruppe:
      Raumtemperatur;
      Beleuchtungsstärke;
      Geräusche;
      Belüftung;
      Luftfeuchte.
A8. Verfahren nach einem der vorhergehenden Aspekte, wonach die Initiierung der Zustandsänderung der Simulationsumgebung für eine bestimmte medizinische Situation charakteristisch ist.
A9. Verfahren nach einem der Aspekte A2 bis A8, wonach der künstliche Ersatzpatient menschliche Gestalt hat und mit mindestens einer der folgenden Vorrichtungen ausgestattet ist:
   künstlichen Augen, die derart ansteuerbar sind, dass ihr optisches Erscheinungsbild veränderbar ist;
   einem Lautsprecher;
   mindestens einem Aktor, der zum Bewegen mindestens eines Teils des künstlichen Ersatzpatienten eingerichtet ist;
   mindestens ein Aktor, der derart im künstlichen Ersatzpatienten angeordnet und konfiguriert ist, dass durch die Ansteuerung des Aktors ein fühlbarer Puls erzeugt wird.
A10. Verfahren nach Aspekt A9, wobei die Ansteuerung auswählbar ist aus der folgenden Gruppe:
   Ansteuern der künstlichen Augen derart, dass deren Erscheinungsbild einem auswählbaren Zustand des künstlichen Ersatzpatienten entspricht;
   Ansteuern des Lautsprechers derart, dass über ihn menschliche Laute ausgegeben werden, die einem auswählbaren Zustand des künstlichen Ersatzpatienten entspricht;
   Ansteuern des mindestens einen Aktors, der zum Bewegen des mindestens einen Teils des künstlichen Ersatzpatienten eingerichtet ist, derart, dass eine auswählbare Bewegung des künstlichen Ersatzpatienten erfolgt;
   Ansteuern des mindestens einen Aktors, der derart im künstlichen Ersatzpatienten angeordnet und konfiguriert ist, dass durch die Ansteuerung des Aktors ein fühlbarer Puls erzeugt wird, derart, dass der somit erzeugte Puls einem auswählbaren Zustand des künstlichen Ersatzpatienten entspricht.
A11. Verfahren einem der Aspekte A2 bis A10, wonach der künstliche Ersatzpatient mit einem Flüssigkeitssystem ausgestattet ist, das zumindest eine Flüssigkeitsleitung aufweist, die mit einem externen Flüssigkeitssystem verbindbar ist, und wobei das Flüssigkeitssystem des künstlichen Ersatzpatienten derart ausgestaltet ist, dass der Fluss und der Flüssigkeitsdruck in der zumindest einen Flüssigkeitsleitung unabhängig voneinander beeinflussbar sind.
A12. Verfahren nach Aspekt A11, wonach der Fluss und/oder der Flüssigkeitsdruck in der zumindest einen Flüssigkeitsleitung einem auswählbaren Zustand des künstlichen Ersatzpatienten entsprechen.
A13. Verfahren nach einem der vorhergehenden Aspekte, wonach die aufgezeichneten Simulationsdaten zur Bewertung der Qualifikation einer Testperson heran gezogen werden.
A14. Verfahren nach einem der vorhergehenden Aspekte, wonach die aufgezeichneten Simulationsdaten zur Bewertung der Bedienbarkeit des medizintechnischen Geräts heran gezogen werden.
A15. Verfahren nach einem der vorhergehenden Aspekte, wonach das medizintechnische Gerät ein Blutbehandlungsgerät ist und insbesondere zur Dialyse eingerichtet ist.
A16. Verfahren nach einem der vorhergehenden Aspekte, wonach das Abbruchkriterium das Eintreten einer schädlichen Situation ist.
A17: Verfahren nach einem der vorhergehenden Aspekte, wonach die Initiierung der Zustandsänderung der Simulationsumgebung für eine bestimmte medizinische Situation charakteristisch ist.
B1. System umfassend:
   einem medizintechnischem Gerät als Teil einer Simulationsumgebung, dessen Betriebsparameter steuer-und beobachtbar sind;
   mindestens einer Vorrichtung zum Aufzeichnen von Simulationsdaten, wenigstens für die Dauer der Simulation;
   mindestens einem Personensensor, der dazu eingerichtet ist, Daten, die eine Testperson betreffen, die sich in Simulationsraum, der Teil der Simulationsumgebung ist, befindet, zu erfassen,
   gekennzeichnet durch
   mindestens eine Steuervorrichtung, die datentechnisch mit dem medizintechnischen Gerät und dem mindestens einen Personensensor verbunden ist; wobei die mindestens eine Steuervorrichtung dazu eingerichtet ist, eine Zustandsänderung der Simulationsumgebung zu initiieren.
B2. System nach Aspekt B1 umfassend
   einen künstlichen Ersatzpatienten als Teil der Simulationsumgebung, wobei der künstliche Ersatzpatient mit dem medizintechnischen Gerät zum Zweck einer simulierten Behandlungsausführung interagieren kann und dessen Betriebsparameter steuer- und beobachtbar sind, und der mit der mindestens einen Steuervorrichtung datentechnisch verbindbar ist.
B3. System nach einem der vorhergehenden Aspekte B1-B2, wonach der mindestens eine Personensensor dazu eingerichtet ist, Parameter zu detektieren, die ausgewählt sein können aus der folgenden Gruppe:
   physiologische Daten einer Testperson;
   Fokussierpunkte einer Testperson;
   Audio- und/oder Videoaufzeichnungen einer Testperson;
   Eingaben einer Testperson in eine Bedienerschnittstelle.
B4. System nach einem der Aspekte B1 - B3, wonach die Simulationsdaten die Betriebsparameter des medizintechnischen Geräts und/oder die Betriebsparameter des künstlichen Ersatzpatienten umfassen.
B5. System nach einem der Aspekte B1 - B4, wonach die mindestens eine Steuervorrichtung so konfiguriert ist, dass sie bei einer Initiierung einer Zustandsänderung der Simulationsumgebung die folgenden Schritte ausführt:
   Ansteuern des medizintechnischen Geräts und/oder einer mit dem medizintechnischen Gerät interagierenden Vorrichtung über die datentechnische Verbindung derart, dass das medizintechnische Gerät und/oder die mit dem medizintechnischen Gerät interagierende Vorrichtung seinen momentanen Betriebszustand in einen anderen Betriebszustand überführt.
B6. System nach Aspekt B2, wonach die mindestens eine Steuervorrichtung so konfiguriert ist, dass sie bei einer Initiierung einer Zustandsänderung der Simulationsumgebung die folgenden Schritte ausführt:
   Ansteuern des künstlichen Ersatzpatienten über die datentechnische Verbindung derart, dass der künstliche Ersatzpatient seinen momentanen Betriebszustand in einen anderen Betriebszustand überführt.
B7. System nach einem der Aspekte B1 - B6, wonach die mindestens eine Steuervorrichtung datentechnisch mit Vorrichtungen zum Einstellen von Umweltvariablen der Simulationsumgebung verbunden ist, die so durch die Steuervorrichtung einstellbar sind, und die mindestens eine Steuervorrichtung so konfiguriert ist, dass sie bei einer Initiierung einer Zustandsänderung der Simulationsumgebung den folgenden Schritt ausführt:
   Ansteuern und Einstellen der Vorrichtungen zum Einstellen von Umweltvariablen.
   wobei die Umweltvariablen des Simulationsraums ausgewählt sein können aus einer Gruppe umfassend:
      Raumtemperatur;
      Beleuchtungsstärke;
      Geräusche;
      Belüftung;
      Luftfeuchte.
B8. System nach einem der Aspekte B2 bis B8, wonach der künstliche Ersatzpatient menschliche Gestalt hat und mit mindestens einer der folgenden Vorrichtungen ausgestattet ist:
   künstlichen Augen, die derart ansteuerbar sind, dass ihr optisches Erscheinungsbild veränderbar ist;
   einem Lautsprecher;
   mindestens einen Aktor, der zum Bewegen mindestens eines Teils des künstlichen Ersatzpatienten eingerichtet ist;
   mindestens einem Aktor, der derart im künstlichen Ersatzpatienten angeordnet und konfiguriert ist, dass durch die Ansteuerung des Aktors ein fühlbarer Puls erzeugt wird.
B9. System nach einem der Aspekte B2 bis B8, wonach der künstliche Ersatzpatient mit einem Flüssigkeitssystem ausgestattet ist, das zumindest eine Flüssigkeitsleitung aufweist, das mit einem externen Flüssigkeitssystem verbindbar ist und wobei das Flüssigkeitssystem des künstlichen Ersatzpatienten derart ausgestaltet ist, dass der Fluss und der Flüssigkeitsdruck in der zumindest einen Flüssigkeitsleitung unabhängig voneinander beeinflussbar sind.
B10. System nach einem der Aspekte B2 bis B9, wonach die Interaktion des künstlichen Ersatzpatienten mit dem medizintechnischen Gerät eine simulierte Behandlungsausführung umfasst, wobei Mittel vorgesehen sind, die zum Flüssigkeitstransport zumindest einer Flüssigkeit zwischen dem medizintechnischem Gerät und dem künstlichen Ersatzpatienten eingerichtet sind.
B11. System nach Aspekt B10, umfassend eine Vorrichtung zum steuerbaren Unterbinden oder Behindern des Flüssigkeitstransports.
B12. System nach Aspekt B5 oder B6, umfassend einen Computer mit Bedienerschnittstelle, der dazu konfiguriert und programmiert ist, eine Vielzahl auswählbarer Ursachen für eine bestimmte medizinische Situation, die dem momentanen Betriebszustand des medizintechnischen Geräts oder dem momentanen Betriebszustand der mit dem medizintechnischen Gerät interagierenden Vorrichtung oder dem momentanen Betriebszustand des künstlichen Ersatzpatienten entspricht, auf der Bedienerschnittstelle anzuzeigen und eine Eingabe der Testperson, die einer ausgewählten und angezeigten Ursache für diese medizinische Situation entspricht, zu empfangen und datentechnisch zu verarbeiten.
B13. System nach einem der Aspekte B1 - B12, wonach das medizintechnische Gerät ein Blutbehandlungsgerät ist und insbesondere zur Dialyse eingerichtet ist.
B14. Softwareprogramm oder Softwareprogrammprodukt, zum Betrieb des Systems nach Aspekt B12, mit auf einem Datenträger gespeicherten, von einem Computer lesbaren und ausführbaren Programminstruktionen, die, wenn sie von dem Computer ausgeführt werden, den Computer dazu programmieren, eine Vielzahl auswählbarer Ursachen für die bestimmte medizinische Situation auf der Bedienerschnittstelle anzuzeigen und eine Eingabe der Testperson, die einer ausgewählten und angezeigten Ursache entspricht, zu empfangen und datentechnisch zu verarbeiten.
B15. Softwareprogramm oder Softwareprogrammprodukt, zum Betrieb des Systems nach einem der Aspekte B1 - B13, mit auf einem Datenträger gespeicherten, von der mindestens eine Steuervorrichtung lesbaren und ausführbaren Programminstruktionen, die, wenn sie von der mindestens eine Steuervorrichtung ausgeführt werden, die mindestens eine Steuervorrichtung dazu programmieren, die Zustandsänderung der Simulationsumgebung zu initiieren und/oder die aufgezeichneten Simulationsdaten zu verarbeiten und/oder die vom mindestens einen Personensensor erfassten Daten zu verarbeiten.
C1. Medizintechnisches Gerät umfassend mindestens einer Vorrichtung zur steuerbaren Herbeiführung eines abnormalen Betriebszustands des medizintechnischen Geräts.
C2. Medizintechnisches Gerät nach Aspekt C1, wobei die mindestens eine Vorrichtung zur steuerbaren Herbeiführung eines abnormalen Betriebszustands umfasst: mindestens eine erste Steuervorrichtung, mindestens einen Aktor und mindestens einem Sensor und eine Datenschnittstelle, die zum Datenaustausch mit einer zweiten Steuervorrichtung eingerichtet ist,
   dadurch gekennzeichnet, dass
   das medizintechnische Gerät dazu eingerichtet ist, in einem Betriebsmodus den mindestens einen Aktor und den mindestens einen Sensor von der zweiten Steuervorrichtung ansteuer- und kontrollierbar zu machen.
C3. Medizintechnisches Gerät nach einem der Aspekte C1 - C2, wonach das medizintechnische Gerät ein Blutbehandlungsgerät ist und insbesondere zur Dialyse eingerichtet ist.
C4. Medizintechnisches Gerät nach Aspekt C3, das mit Einwegartikeln zur Ausbildung eines extrakorporalen Blutkreislaufs aufrüstbar ist, wonach die mindestens eine Vorrichtung zur steuerbaren Herbeiführung eines abnormalen Betriebszustands aus der nachfolgenden Gruppe ausgewählt sein kann:
   Mittel zum Einbringen von Luft in den extrakorporalen Blutkreislauf;
   Mittel zur Unterbrechung des extrakorporalen Blutkreislaufs;
   Mittel zum Absondern von Flüssigkeit außerhalb des extrakorporalen Blutkreislaufs;
   Mittel zur Unterbrechung der Stromversorgung des Blutbehandlungsgeräts.
C5. Medizintechnisches Gerät nach Aspekt C4, wobei das Mittel zum Einbringen von Luft in den extrakorporalen Blutkreislauf eine Pumpe ist.
C6. Medizintechnisches Gerät nach Aspekt C4, wobei das Mittel zur Unterbrechung des extrakorporalen Blutkreislaufs eine elektromechanische, pneumatische oder hydraulische Klemme ist.
C7. Medizintechnisches Gerät nach Aspekt C4, wobei das Mittel zum Absondern von Flüssigkeit außerhalb des extrakorporalen Blutkreislaufs eine Pumpe umfasst oder ein Ventil ist, das Flüssigkeit aus dem extrakorporalen Blutkreislauf abzweigt.
C8. Medizintechnisches Gerät nach einem der Aspekte C1 - C7 mit einem künstlichen Ersatzpatienten, der mit dem medizintechnischen Gerät im Rahmen einer Behandlungsausführung interagiert.
C9. Medizintechnisches Gerät nach Aspekt C4 mit zur Ausbildung eines extrakorporalen Blutkreislaufs aufgerüsteten Einmalartikeln.
C10. Medizintechnisches Gerät nach Aspekt C9, wobei der extrakorporale Blutkreislauf mit einer Blutersatzflüssigkeit gefüllt ist.
C11. Medizintechnisches Gerät nach einem der Aspekte C9 oder C10 mit einem künstlichen Ersatzpatienten, wobei der künstliche Ersatzpatient mit einem Flüssigkeitssystem ausgestattet ist, das zumindest eine Flüssigkeitsleitung aufweist und mit dem extrakorporalen Blutkreislauf verbunden ist.
C12. Medizintechnisches Gerät nach einem der Aspekte C1 - C11 mit zumindest einer Bedienerschnittstelle, wobei das medizintechnische Gerät dazu konfiguriert und programmiert ist, eine Vielzahl auswählbarer Ursachen für den herbeigeführten abnormalen Betriebszustand auf der Bedienerschnittstelle anzuzeigen und eine Bedienereingabe, die der Auswahl eine der angezeigten Ursache entspricht, zu empfangen und datentechnisch zu verarbeiten.
C13. Medizintechnisches Gerät nach Aspekt C12, umfassend eine Kamera, ein Mikrofon und einen Lautsprecher, wobei das medizintechnische Gerät dazu eingerichtet ist, eine audiovisuelle Kommunikation mit einem entfernten Gerät aufzubauen.
C14. Softwareprogramm oder Softwareprogrammprodukt, zum Betrieb des medizintechnischen Geräts nach einem Aspekte C12 oder C13, mit auf einem Datenträger gespeicherten, von der mindestens eine Steuervorrichtung lesbaren und ausführbaren Programminstruktionen, die, wenn sie von der mindestens eine Steuervorrichtung ausgeführt werden, die mindestens eine Steuervorrichtung dazu programmieren, eine Vielzahl auswählbarer Ursachen für den herbeigeführten abnormalen Betriebszustand auf der Bedienerschnittstelle anzuzeigen und eine Bedienereingabe, die der Auswahl eine der angezeigten Ursache entspricht, zu empfangen und datentechnisch zu verarbeiten.
C15. Verwenden eines medizintechnischen Geräts nach einem der Aspekte C1 -
C14 zum Ausführen eines Verfahrens zur Simulation medizinischer Behandlungen.
D1. Flüssigkeitssystem zur Aufnahme einer medizinischen Flüssigkeit umfassend: eine erste Fluidleitung und eine zweite Fluidleitung, wobei die erste Fluidleitung und die zweite Fluidleitung mit zumindest einer externen Fluidleitung verbindbar sind, dadurch gekennzeichnet
   dass das Flüssigkeitssystem Aktoren aufweist, mit denen der jeweilige Fluss und der jeweilige Flüssigkeitsdruck in der ersten Fluidleitung und in der zweiten Fluidleitung unabhängig voneinander einstellbar sind.
D2. Flüssigkeitssystem nach Aspekt D1 umfassend ein Flüssigkeitsreservoir mit einem Zulauf und einem Ablauf, wobei der Zulauf und der Ablauf über ein erstes Ventil miteinander verbindbar sind, und in dem durch die Verbindung zwischen Zulauf und Ablauf entstehendem ersten Fluidkreislauf eine Flüssigkeitspumpe angeordnet ist, die zum Zirkulieren der Flüssigkeit im ersten Fluidkreislauf eingerichtet ist, und wobei flussaufwärts des ersten Ventils eine Fluidverbindung zur ersten Fluidleitung besteht und flussabwärts des ersten Ventils eine Fluidverbindung zur zweiten Fluidleitung besteht.
D3. Flüssigkeitssystem nach einem der Aspekte D1 - D2, wobei die erste und die zweite Fluidleitung durch ein zweites Ventil miteinander verbindbar sind.
D4. Flüssigkeitssystem nach einem der Aspekte D1 - D3, wonach in der ersten Fluidleitung ein erstes 3-Wegeventil angeordnet ist, dass in einem ersten Betriebsmodus das flussaufwärts des ersten 3-Wegeventils liegende Teilstück der ersten Fluidleitung mit dem flussabwärts des ersten 3-Wegeventils liegende Teilstück der ersten Fluidleitung verbindet und in einem zweiten Betriebsmodus das flussabwärts des ersten 3-Wegeventils liegende Teilstück der ersten Fluidleitung mit der Umgebungsluft verbindet.
D5. Flüssigkeitssystem nach einem nach einem der Aspekte D1 - D4, wonach das Flüssigkeitsreservoir einen Überlauf aufweist, der im Normalbetrieb oberhalb des Flüssigkeitspegels im Flüssigkeitsreservoir angeordnet ist, und wobei vom Überlauf eine Fluidleitung zu einem zweiten 3-Wegeventil führt, das in der zweiten Fluidleitung angeordnet ist, wobei das zweite 3-Wegeventil in einem ersten Betriebsmodus das flussaufwärts des zweiten 3-Wegeventils liegende Teilstück der zweiten Fluidleitung mit dem flussabwärts des zweiten 3-Wegeventils liegende Teilstück der zweiten Fluidleitung verbindet und in einem zweiten Betriebsmodus das flussabwärts des zweiten 3-Wegeventils liegende Teilstück der zweiten Fluidleitung mit der Fluidleitung, die vom Überlauf zum zweiten 3-Wegeventil führt, verbindet.
D6. Flüssigkeitssystem nach einem nach einem der Aspekte D1 - D5, wonach in der ersten Flüssigkeitsleitung und in der zweiten Flüssigkeitsleitung jeweils ein Mittel zur Einstellung des Flusses angeordnet ist.
D7. Flüssigkeitssystem nach einem der Aspekte D1 bis D6, wonach in dem ersten Fluidkreislauf ein Mittel zur Einstellung des Flusses angeordnet ist.
D8. Flüssigkeitssystem einem nach einem der Aspekte D1 - D7, wonach in dem ersten Fluidkreislauf eine Tropfkammer angeordnet ist.
D9. Flüssigkeitssystem nach einem der Aspekte D1 - D8, wonach Mittel vorgesehen sind, um die Viskosität der Flüssigkeit, die sich in den Fluidleitungen befindet, zu beeinflussen.
D10. Flüssigkeitssystem nach einem der Aspekte D1 - D9, wonach zumindest die erste und die zweite Fluidleitung innerhalb eines künstlichen menschlichen Arms verlaufen.
D11. Flüssigkeitssystem nach Aspekt D10, wonach zur Verbindung der ersten und/oder zweiten Fluidleitung mit zumindest einer externen Fluidleitung zumindest eine Punktionsstelle am künstlichen menschlichen Arm vorgesehen ist und an dieser zumindest einen Punktionsstelle zumindest eine optische Signalisiervorrichtung vorgesehen ist.
D12. Flüssigkeitssystem nach einem der Aspekte D10 oder D11, wonach der künstliche menschliche Arm mit einem Aktor ausgestattet ist, der derart konfiguriert ist, dass durch die Ansteuerung des Aktors ein fühlbarer Puls erzeugt wird.
D13. Flüssigkeitssystem einem nach einem der Aspekte D1 - D12, wonach die Verbindung der ersten Fluidleitung und der zweiten Fluidleitung mit der zumindest einen externen Fluidleitung durch Hohlnadeln herstellbar ist, die durch Aktoren derart verfahrbar sind, dass die jeweilige Hohlnadel aus der ersten oder zweiten Fluidleitung herausrutscht, oder dass die jeweilige Hohlnadel zur inneren Begrenzungsfläche der verbundenen Fluidleitung bewegt wird.
D14. Flüssigkeitssystem nach einem nach einem der Aspekte D1 - D13, wonach die Aktoren durch eine Steuervorrichtung ansteuerbar und/oder auslesbar sind und/oder Sensoren vorgesehen sind, die Sensoren zur Bestimmung des Flusses und/oder des Flüssigkeitsdrucks im Flüssigkeitssystem eingerichtet sind, und wobei die Aktoren und die Sensoren durch eine Steuervorrichtung ansteuerbar und/oder auslesbar sind.
D15. Flüssigkeitssystem nach Aspekt D1 weiterhin umfassend ein Flüssigkeitsreservoir mit einem Zulauf und einem Ablauf, wobei der Zulauf und der Ablauf über ein erstes Ventil miteinander verbindbar sind und in dem durch die Verbindung zwischen Zulauf und Ablauf entstehendem ersten Fluidkreislauf eine Flüssigkeitspumpe angeordnet ist, die zum Zirkulieren der Flüssigkeit im ersten Fluidkreislauf eingerichtet ist, und wobei flussaufwärts des ersten Ventils eine Fluidverbindung zur ersten Fluidleitung besteht und flussabwärts des ersten Ventils eine Fluidverbindung zur zweiten Fluidleitung besteht.
D16. Flüssigkeitssystem nach Aspekt D15, wonach die Pumpe eine Zentrifugalpumpe ist.
D17. Flüssigkeitssystem nach Aspekt D9, wonach die Flüssigkeit eine magnetorheologische und/oder elektrorheologische Flüssigkeit ist und die Mittel zur Beeinflussung der Viskosität der Flüssigkeit dazu eingerichtet sind, ein magnetisches oder elektrisches Feld in der Flüssigkeit aufzubauen.
D18: Flüssigkeitssystem nach Aspekt D9, wonach die Mittel, um die Viskosität der Flüssigkeit, die sich in den Fluidleitungen befindet, zu beeinflussen, dazu eingerichtet sind, der Flüssigkeit Mittel zuzuführen, die die Viskosität der Flüssigkeit beeinflussen.
D19: Flüssigkeitssystem nach Aspekt D18, wonach die der Flüssigkeit zugeführten Mittel ausgewählt sein können unter den folgenden Stoffen:
   Hydroxyethylstärke;
   Polyethylenglycol;
   Wasser.
D19. Verwendung eines Flüssigkeitssystems nach einem der Aspekte D1 - D14 zum Ausführen eines Verfahrens zur Simulation medizinischer Behandlungen.
E1. Vorrichtung zur Nachbildung zumindest einer menschlichen Eigenschaft, wobei die Nachbildung der zumindest einen menschlichen Eigenschaft von einer Steuervorrichtung kontrollierbar und/oder ansteuerbar ist, dadurch gekennzeichnet, dass
   die menschliche Eigenschaft das vaskuläre System ist, wobei die Nachbildung des vaskulären System umfasst: ein Flüssigkeitssystem zur Aufnahme einer medizinischen Flüssigkeit mit einer ersten Fluidleitung und einer zweiten Fluidleitung, wobei die erste Fluidleitung und die zweite Fluidleitung mit zumindest einer externen Fluidleitung verbindbar sind, und wobei das Flüssigkeitssystem Aktoren aufweist, mit denen der jeweilige Fluss und der jeweilige Flüssigkeitsdruck in der ersten Fluidleitung und in der zweiten Fluidleitung unabhängig voneinander einstellbar sind,
   und/oder
   dass die menschliche Eigenschaft das Erscheinungsbild menschlicher Augen ist, wobei die Nachbildung des Erscheinungsbilds der menschlichen Augen umfasst: künstliche Augen, die derart ansteuerbar sind, dass ihr optisches Erscheinungsbild veränderbar ist.
E2. Vorrichtung nach Aspekt E1, wonach mindestens eine zusätzliche menschliche Eigenschaft ausgewählt sein kann aus der folgenden Gruppe:
   menschliche Lautäußerungen;
   körperliche Bewegung;
   menschlicher Puls;
   Viskosität der Flüssigkeit im vaskulären System;
   Temperatur der Flüssigkeit im vaskulären System;
   Atmung;
   und wobei die Nachbildung der menschlichen Lautäußerungen umfasst: einen Lautsprecher, über den menschliche Lautäußerungen ausgebbar sind,
   und wobei die Nachbildung der körperlichen Bewegung umfasst, mindestens einen Aktor, der zum Bewegen mindestens eines Teils des künstlichen Ersatzpatienten eingerichtet ist,
   und wobei die Nachbildung des menschlichen Puls umfasst, mindestens ein Aktor, der derart im künstlichen Ersatzpatienten angeordnet und konfiguriert ist, dass durch die Ansteuerung des Aktors ein fühlbarer Puls erzeugt wird
   und wobei die Nachbildung der Viskosität der Flüssigkeit im vaskulären System Mittel umfasst, mit denen die Viskosität der Flüssigkeit im Flüssigkeitssystem beeinflussbar ist,
   und wobei die Nachbildung der Temperatur der Flüssigkeit im vaskulären System umfasst, dass eine Heiz-/Kühlvorrichtungen vorgesehen ist, mit der die Temperatur der Flüssigkeit im Flüssigkeitssystem beeinflussbar ist,
   und wobei die Nachbildung der Atmung umfasst, dass der Aktor, der zum Bewegen mindestens eines Teils des künstlichen Ersatzpatienten eingerichtet ist, zum Bewegen des Brustkorbs des künstlichen Ersatzpatienten eingerichtet ist und derart angesteuert wird, dass die erzeugte Bewegung den Brustkorb periodisch hebt und senkt..
E3. Vorrichtung nach Aspekt E2, wonach die Mittel, mit denen die Viskosität der Flüssigkeit im Flüssigkeitssystem beeinflussbar ist, umfassen:
   dass die Flüssigkeit im Flüssigkeitssystem eine magnetorheologische und/oder elektrorheologische Flüssigkeit ist und Mittel vorgesehen sind, die dazu eingerichtet sind, ein magnetisches oder elektrisches Feld in der Flüssigkeit aufzubauen,
   oder dass Mittel vorgesehen sind, die dazu eingerichtet sind, der Flüssigkeit Stoffe zuzuführen, die die Viskosität der Flüssigkeit beeinflussen.
E4. Vorrichtung nach einem der Aspekte E1 - E3, wonach die Vorrichtung die Gestalt eines menschlichen Körpers hat, und wonach zumindest die erste und die zweite Fluidleitung innerhalb eines künstlichen menschlichen Arms der Vorrichtung verlaufen.
E5. Vorrichtung nach Aspekt E4, wonach die Gestalt des menschlichen Körpers unterschiedliche Ausprägungen aufweisen kann, wobei die Ausprägungen folgende Merkmale betreffen können:
   Alter;
   Geschlecht;
   Gewicht;
   Rasse;
   Größe.
E6. Vorrichtung nach einem der Aspekte E1 - E5, wonach zur Verbindung der ersten und/oder zweiten Fluidleitung mit zumindest einer externen Fluidleitung zumindest eine Punktionsstelle vorgesehen ist und an dieser zumindest einen Punktionsstelle zumindest eine optische Signalisiervorrichtung vorgesehen ist.
E7. Vorrichtung nach einem der Aspekte E1 - E5, wonach die künstlichen Augen Monitore oder die Projektion eines Augenbildes umfassen.
E8. Vorrichtung nach Aspekt E1, wonach die künstlichen Augen als Vorrichtung in Brillenform ausgeführt sind, wobei anstelle der Brillengläser ansteuerbare Monitore angebracht sind, und wobei die Monitore derart angesteuert werden können, dass auf ihnen die Darstellung menschlicher Augen angezeigt wird.
E9. Verfahren zur Simulation eines Zustands einer menschlichen Eigenschaft, umfassend die folgenden Schritte:
   Verwenden einer Vorrichtung nach einem der Aspekte E1 - E8;
   Kontrolle der Nachbildung der menschlichen Eigenschaft derart, dass die Nachbildung einem auswählbaren Zustand der menschlichen Eigenschaft entspricht.
E10. Verfahren nach Aspekt E9, wonach der Zustand der menschlichen Eigenschaft ausgewählt sein kann aus:
   Druck und Fluss der Flüssigkeit im vaskulären System;
   Viskosität der Flüssigkeit im vaskulären System;
   Temperatur im vaskulären System;
   Bewegung zumindest eines Teils des menschlichen Körpers;
E11. Verfahren nach Aspekt E9, wonach die menschliche Eigenschaft auswählbar ist aus den folgenden Eigenschaften:
   Geisteszustand;
   Alter;
   Wohlbefinden;
   und wobei die Kontrolle der künstlichen Augen, und/oder des mindestens einen Aktors, der zum Bewegen mindestens eines Teils des künstlichen Ersatzpatienten eingerichtet ist, und/oder des Lautsprechers derart erfolgt, dass die Kontrolle der ausgewählten menschlichen Eigenschaft entspricht.
E12. Verwendung einer Vorrichtung nach einem der Aspekte E1 bis E8 in einem System zur Simulation einer medizinischen Behandlung.
E13. Verfahren nach Aspekt E12, wobei die Vorrichtung mit einem medizintechnischen Gerät interagiert und die Kontrolle der Nachbildung der zumindest einen menschliche Eigenschaft derart geschieht, dass der Zustand der zumindest einen menschlichen Eigenschaft einer auswählbaren medizinischen Situation entspricht.
E14. Verfahren nach Aspekt E13, wobei das medizintechnische Gerät ein Blutbehandlungsgerät ist und insbesondere zur Dialyse eingerichtet ist.
E15. Softwareprogramm oder Softwareprogrammprodukt, das auf einem Datenträger gespeichert ist, dadurch gekennzeichnet, dass wenn es von der Steuervorrichtung nach einem der Aspekte E1 bis E8 betrieben wird, die Steuervorrichtung so programmiert, dass die Verfahren nach einem der Aspekten E9 bis E14 ausgeführt werden.

## Patentansprüche

1. Verfahren zur Simulation medizinischer Behandlungen mit den Schritten:
Bereitstellen mindestens eines medizintechnischen Geräts als Teil einer Simulationsumgebung, dessen Betriebsparameter steuer-und beobachtbar sind;
Aufzeichnen von Simulationsdaten, wenigstens für die Dauer der Simulation,
Initiierung einer Zustandsänderung der Simulationsumgebung,
Überprüfen eines Abbruchkriteriums.

2. Verfahren nach Anspruch 1 mit dem zusätzlichen Schritt:
Bereitstellen eines künstlichen Ersatzpatienten als Teil der Simulationsumgebung, wobei der künstliche Ersatzpatient mit dem medizintechnischen Gerät zum Zweck einer simulierten Behandlungsausführung interagieren kann und dessen Betriebsparameter steuer- und beobachtbar sind.

3. Verfahren nach Anspruch 2, wonach die Simulationsdaten Betriebsparameter des künstlichen Ersatzpatienten umfassen.

4. Verfahren nach einem der vorhergehenden Ansprüche, wonach die Simulationsdaten ausgewählt sein können aus der folgenden Gruppe:
physiologische Daten einer Testperson;
Fokussierpunkte einer Testperson;
Audio- und/oder Videoaufzeichnungen einer Testperson;
Eingaben einer Testperson in eine Bedienerschnittstelle;
Betriebsparameter des medizintechnischen Geräts.

5. Verfahren nach einem der vorhergehenden Ansprüche, wonach die Initiierung der Zustandsänderung der Simulationsumgebung umfasst:
Ansteuern des medizintechnischen Geräts und/oder einer mit dem medizintechnischen Gerät interagierenden Vorrichtung derart, dass das medizintechnische Gerät und/oder die mit dem medizintechnischen Gerät interagierende Vorrichtung seinen momentanen Betriebszustand in einen anderen Betriebszustand überführt.

6. Verfahren nach Anspruch 2, wonach die Initiierung der Zustandsänderung der Simulationsumgebung umfasst:
Ansteuern des künstlichen Ersatzpatienten derart, dass der künstliche Ersatzpatient seinen momentanen Betriebszustand in einen anderen Betriebszustand überführt.

7. Verfahren nach einem der vorhergehenden Ansprüche, wonach die Initiierung der Zustandsänderung der Simulationsumgebung umfasst:
Ändern der Umweltvariablen des Simulationsraums, wobei die Umweltvariablen des Simulationsraums ausgewählt sind aus der Gruppe:
Raumtemperatur;
Beleuchtungsstärke;
Geräusche;
Belüftung;
Luftfeuchte.

8. Verfahren nach einem der vorhergehenden Ansprüche, wonach die Initiierung der Zustandsänderung der Simulationsumgebung für eine bestimmte medizinische Situation charakteristisch ist.

9. Verfahren nach einem der Ansprüche 2 bis 8, wonach der künstliche Ersatzpatient menschliche Gestalt hat und mit mindestens einer der folgenden Vorrichtungen ausgestattet ist:
künstlichen Augen, die derart ansteuerbar sind, dass ihr optisches Erscheinungsbild veränderbar ist;
einem Lautsprecher;
mindestens einem Aktor, der zum Bewegen mindestens eines Gliedmaßes des künstlichen Ersatzpatienten eingerichtet ist;
mindestens einem Aktor, der derart im künstlichen Ersatzpatienten angeordnet und konfiguriert ist, dass durch die Ansteuerung des Aktors ein fühlbarer Puls erzeugt wird.

10. Verfahren nach Anspruch 9, wobei die Ansteuerung auswählbar ist aus der folgenden Gruppe:
Ansteuern der künstlichen Augen derart, dass deren Erscheinungsbild einem auswählbaren körperlichen Zustand des künstlichen Ersatzpatienten entspricht;
Ansteuern des Lautsprechers derart, dass über ihn menschliche Laute ausgegeben werden, die einem auswählbaren körperlichen Zustand des künstlichen Ersatzpatienten entsprechen;
Ansteuern des mindestens einen Aktors, der zum Bewegen des mindestens einen Gliedmaß des künstlichen Ersatzpatienten eingerichtet ist, derart, dass eine auswählbare Bewegung des künstlichen Ersatzpatienten erfolgt;
Ansteuern des mindestens einen Aktors, der derart im künstlichen Ersatzpatienten angeordnet und konfiguriert ist, dass durch die Ansteuerung des Aktors ein fühlbarer Puls erzeugt wird, derart, dass der somit erzeugte Puls einem auswählbaren körperlichen Zustand des künstlichen Ersatzpatienten entspricht.

11. Verfahren einem der Ansprüche 2 bis 10, wonach der künstliche Ersatzpatient mit einem Flüssigkeitssystem ausgestattet ist, das zumindest eine Flüssigkeitsleitung aufweist, die mit einem externen Flüssigkeitssystem verbindbar ist und wobei das Flüssigkeitssystem des künstlichen Ersatzpatienten derart ausgestaltet ist, dass der Fluss und der Flüssigkeitsdruck in der zumindest einen Flüssigkeitsleitung unabhängig voneinander beeinflussbar sind.

12. Verfahren nach Anspruch 11, wonach der Fluss und/oder der Flüssigkeitsdruck in der zumindest einen Flüssigkeitsleitung einem auswählbaren körperlichen Zustand des künstlichen Ersatzpatienten entsprechen.

13. Verfahren nach einem der vorhergehenden Ansprüche, wonach die aufgezeichneten Simulationsdaten zur Bewertung der Qualifikation einer Testperson heran gezogen werden.

14. Verfahren nach einem der vorhergehenden Ansprüche, wonach die aufgezeichneten Simulationsdaten zur Bewertung der Bedienbarkeit des medizintechnischen Geräts heran gezogen werden.

15. Verfahren nach einem der vorhergehenden Ansprüche, wonach das medizintechnische Gerät ein Blutbehandlungsgerät ist und insbesondere zur Dialyse eingerichtet ist.
